(19) Europäisches Patentamt
European Patent Office
Office européen des brevets

(11) **EP 4 439 048 A1**

(12) **EUROPEAN PATENT APPLICATION**
published in accordance with Art. 153(4) EPC

(43) Date of publication:
**02.10.2024 Bulletin 2024/40**

(21) Application number: **22898646.9**

(22) Date of filing: **25.11.2022**

(51) International Patent Classification (IPC):
**G01N 21/3504** *(2014.01)*

(52) Cooperative Patent Classification (CPC):
**G01N 21/3504**

(86) International application number:
**PCT/JP2022/043508**

(87) International publication number:
**WO 2023/095864 (01.06.2023 Gazette 2023/22)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **25.11.2021 JP 2021191219**

(71) Applicant: HORIBA, Ltd.
**Kyoto-shi, Kyoto 601-8510 (JP)**

(72) Inventors:
• SHIBUYA, Kyoji
**Kyoto-shi, Kyoto 601-8510 (JP)**
• HAMAUCHI, Shota
**Kyoto-shi, Kyoto 601-8510 (JP)**
• TSUKATANI, Kosuke
**Kyoto-shi, Kyoto 601-8510 (JP)**
• NIINA, Kodai
**Kyoto-shi, Kyoto 601-8510 (JP)**
• IDO, Takuya
**Kyoto-shi, Kyoto 601-8510 (JP)**

(74) Representative: **Müller Hoffmann & Partner
Patentanwälte mbB
St.-Martin-Straße 58
81541 München (DE)**

(54) **ANALYSIS DEVICE, PROGRAM FOR ANALYSIS DEVICE, AND ANALYSIS METHOD**

(57) The present invention enables a concentration of a measurement target component in a processing gas to be accurately measured. The present invention enables a concentration of carbon dioxide to be calculated based on a carbon dioxide absorption between 4.23 ~ 4.24 $\mu$m, or 4.34 ~ 4.35 $\mu$m, a concentration of carbon monoxide to be calculated based on a carbon monoxide absorption between 4.59 ~ 4.61 $\mu$m, a concentration of water to be calculated based on a water absorption between 5.89 ~ 6.12 $\mu$m, a concentration of acetylene to be calculated based on an acetylene absorption between 7.56 ~ 7.66 $\mu$m, or 7.27 ~ 7.81 $\mu$m, a concentration of methane to be calculated based on a methane absorption between 7.67 ~ 7.80 $\mu$m, or 8.10 ~ 8.14 $\mu$m, a concentration of ethylene to be calculated based on an ethylene absorption between 8.46 - 8.60 $\mu$m, a concentration of ethane to be calculated based on an ethane absorption between 6.13 ~ 6.14 $\mu$m, or 6.09 ~ 6.45 $\mu$m, a concentration of ammonia to be calculated based on an ammonia absorption between 6.06 ~ 6.25 $\mu$m, or 8.62 ~ 9.09 $\mu$m, and a concentration of methanol to be calculated based on a methanol absorption between 9.35 ~ 9.62 $\mu$m.

Fig.1

**Description**

[Technical Field]

The present invention relates to an analysis device and the like that is used, for example, in gas component analysis and the like.

[Technical Background]

In a case in which a measurement target component in the form of at least one of carbon dioxide ($CO_2$), carbon monoxide (CO), ethylene ($C_2H_4$), ethane ($C_2H_6$), water ($H_2O$), acetylene ($C_2H_2$), methane ($CH_4$), ammonia ($NH_3$), and methanol ($CH_3OH$) in processing gases including natural gas or the like used in a chemical plant is to be measured, measurement errors are generated by any interference components that are present in the processing gas in addition to the measurement target component. More specifically, an absorption spectrum of an interference component overlaps an absorption peak position of the aforementioned measurement target component, so that errors are generated in the concentration quantification.

On the other hand, the technology described in Patent Document 1 may be considered as a way to correct interference effects from an interference component on a measurement target component.

[Documents of the Prior Art]

[Patent Documents]

[Patent Document 1] Japanese Unexamined Patent Application (JP-A) No. 2016-90521

[Disclosure of the Invention]

[Problems to be Solved by the Invention]

However, even in a case in which the analysis device of Patent Document 1 is used, in order to more effectively reduce interference effects on a measurement target component in a processing gas, it is necessary for a wavelength range in which there is a suitable absorption of the measurement target component to be used for the measurement. To aid in the selection of this wavelength range it is possible, to a certain extent, to perform a preliminary investigation using a publicly available infrared absorption spectrum database such as HITRAN or the like. However, there is a limit as to the types of gases and wavelength ranges for which a database can be used, and it only becomes possible to select a correct wavelength range by performing repeated experiments using the actual measurement target gas and interference gas. Moreover, even if the measurement target component is the same, the appropriate wavelength range may vary due to the concentration, pressure, or temperature of the measurement target component, or due to the type of interference gas that is present as well as the concentration range thereof. In the analysis device described in Patent Document 1 no consideration is given to setting a wavelength range that is suitable for more effectively reducing interference effects, and it cannot be said that by simply employing this analysis device it is possible to effectively remove interference effects on a measurement target component.

The present invention was therefore conceived in view of the above-described problem, and it is a principal object thereof to more effectively reduce interference effects on the concentration of a measurement target component in the form of at least one of carbon dioxide ($CO_2$), carbon monoxide (CO), ethylene ($C_2H_4$), ethane ($C_2H_6$), water ($H_2O$), acetylene ($C_2H_2$), methane ($CH_4$), ammonia ($NH_3$), or methanol ($CH_3OH$ that are present in a processing gas, so as to enable more accurate measurements to be made.

[Means for Solving the Problem]

Namely, an analysis device according to the present invention is an analysis device that measures a concentration of a measurement target component in the form of at least one of carbon dioxide, carbon monoxide, ethylene, ethane, water, acetylene, methane, ammonia, or methanol that are present in a processing gas, and is characterized in being provided with a laser light source that irradiates reference light onto the processing gas, a photodetector that detects an intensity of sample light that is generated as a result of the reference light being transmitted through the processing gas, and a concentration calculation unit that calculates a concentration of the measurement target component based on an output signal from the photodetector, wherein, in a case in which a concentration of the carbon dioxide is being measured, the concentration calculation unit calculates this concentration based on a carbon dioxide absorption between 4.23 ~ 4.24 $\mu$m, or 4.34 ~ 4.35 $\mu$m, in a case in which a concentration of the carbon monoxide is being measured, the concentration calculation unit calculates the concentration of the carbon monoxide between 4.59 - 4.61 $\mu$m, in a case in which a concentration of the water is being measured, the concentration calculation unit calculates the concentration of the water between 5.89 - 6.12 $\mu$m, in a case in which a concentration of the acetylene is being measured, the concentration calculation unit calculates the concentration of the acetylene between 7.56 - 7.66 $\mu$m, or 7.27 - 7.81 $\mu$m, in a case in which a concentration of the methane is being measured, the concentration calculation unit calculates the concentration of the methane between 7.67 - 7.80 $\mu$m, or 8.10 - 8.14 $\mu$m, in a case in which a concentration of the ethylene is being measured, the concentration calculation unit calculates the concentration of the ethylene between 8.46 - 8.60 $\mu$m, in a case in which a concentration of the ethane is being measured, the concentration calculation unit calculates the concentration of the ethane between 6.13 - 6.14 $\mu$m, or 6.09 - 6.45 $\mu$m, in a case in which a concentration of the ammonia is being measured, the concentration calculation unit calculates this concentration based on an ammonia absorption between 6.06 - 6.25 $\mu$m, or 8.62 - 9.09 $\mu$m, and in a case in which a concentration of the methanol is being measured, the concentration calculation unit calculates this concentration based on a methanol absorption between 9.35 ~ 9.62 $\mu$m.

If this type of analysis device is employed, then it becomes possible to accurately measure a concentration of a measurement target component in the form of at least one of carbon dioxide ($CO_2$), carbon monoxide (CO), ethylene ($C_2H_4$), ethane ($C_2H_6$), water ($H_2O$), acetylene ($C_2H_2$), methane ($CH_4$), ammonia ($NH_3$), and methanol ($CH_3OH$) in a processing gas. This is described below in greater detail.

Moreover, the analysis device of the present invention makes it possible to further reduce interference effects by modulating an oscillation wavelength of a laser light source and acquiring an absorption modulation signal or absorption spectrum that are obtained by collecting an absorption signal in each wavelength, and then using a difference in the characteristics of the absorption modulation signals or absorption spectrum between a measurement target component and an interference component. At this time, the wider the wavelength modulation range, the greater the difference in the characteristics of the absorption modulation signals or absorption spectrum between a measurement target component and an interference component that can be obtained. However, the trade-off for this is that, because the proportion of the wavelength modulation range occupied by the absorption peak of the measurement target component is decreased, there is a reduction in the measurement sensitivity. Accordingly, in order to attain a suitable balance, it is desirable that the wavelength modulation range be set between 0.1 - 2 cm$^{-1}$ so as to match the configuration of the absorption modulation signals or absorption spectrum of the measurement target component and interference component.

Moreover, the analysis device of the present invention makes it possible to measure the aforementioned gases at low concentrations of 100 ppm or less by using as a light source a quantum cascade laser that emits mid-infrared range laser light in which these gases display the strongest absorption, so as to create a long optical path length using a multiple reflection cell or a resonance cell. Here, an optical path length of not less than 1 m and not more than 100 m may be used as the long optical path length, with an optical path length of not less than 1 m and not more than 50 m being preferable, an optical path length of not less than 5 m and not more than 30 m being more preferable, and an optical path length of not less than 5 m and not more than 15 m being even more preferable.

In a case in which, using a multiple reflection cell or the like, the analysis device of the present invention measures a concentration of carbon dioxide ($CO_2$) at a low concentration of 100 ppm or less, the analysis device of the present invention calculates this concentration based on a carbon dioxide ($CO_2$) absorption between 4.23 ~ 4.24 $\mu$m. Here, the laser light source emits laser light having an oscillation wavelength that includes a wavelength between 4.23 - 4.24 $\mu$m.

A wavelength between 4.23 - 4.24 $\mu$m, or preferably a wavelength between 4.234 - 4.238 $\mu$m, or more preferably a wavelength of 4.2347 $\mu$m or 4.2371 $\mu$m is a wavelength in which the strongest absorption line of carbon dioxide ($CO_2$) is present, and is where the absorption intensities of methane ($CH_4$), ethylene ($C_2H_4$), and/or ethane ($C_2H_6$), which are interference components in the processing gases of this wavelength region, are small, so that any interference effects from these are small. As a result, it is possible to improve the concentration measurement accuracy when measuring a low concentration of carbon dioxide ($CO_2$) in a processing gas that contains a high concentration of methane ($CH_4$), ethylene ($C_2H_4$), and/or ethane ($C_2H_6$).

In a case in which, using a multiple reflection cell or the like, the analysis device of the present invention measures a concentration of carbon dioxide ($CO_2$) at an intermediate concentration of from 100 ppm to 1%, the analysis device of the present invention calculates this concentration based on a carbon dioxide ($CO_2$) absorption between 4.34 ~ 4.35 $\mu$m. Here, the laser light source emits laser light having an oscillation wavelength that includes a wavelength between 4.34 - 4.35 $\mu$m.

A wavelength between 4.34 - 4.35 $\mu$m, or preferably a wavelength between 4.342 - 4.347 $\mu$m, or more preferably a wavelength of 4.3428 $\mu$m or 4.3469 $\mu$m is a wavelength in which one of the intermediate strength absorption lines of carbon dioxide ($CO_2$) is present, and is where the absorption intensities of methane ($CH_4$), ethylene ($C_2H_4$), and/or ethane ($C_2H_6$), which are interference components in the processing gases of this wavelength region, are small, so that any interference effects from these are small. As a result, it is possible to improve the concentration measurement accuracy when measuring an intermediate concentration of carbon dioxide (COz) in a processing gas that contains a high concentration of methane ($CH_4$), ethylene ($C_2H_4$), and/or ethane ($C_2H_6$).

In a case in which, using a multiple reflection cell or the like, the analysis device of the present invention measures a concentration of carbon monoxide (CO) at a low concentration of 100 ppm or less, the analysis device of the present invention calculates this concentration based on a carbon monoxide (CO) absorption between 4.59 ~ 4.61 $\mu$m . Here, the laser light source emits laser light having an oscillation wavelength that includes a wavelength between 4.59 - 4.61 $\mu$m.

A wavelength between 4.59 - 4.61 $\mu$m, or preferably a wavelength between 4.594 - 4.604 $\mu$m, or more preferably a wavelength of 4.5950 $\mu$m or 4.6024 $\mu$m is a wavelength in which one of the strongest absorption lines of carbon monoxide (CO) is present, and is where the absorption intensities of methane ($CH_4$), ethylene ($C_2H_4$), and/or ethane ($C_2H_6$), which are interference components in the processing gases of this wavelength region, are small, so that any interference effects from these are small. As a result, it is possible to improve the concentration measurement accuracy when measuring a low concentration of carbon monoxide (CO) in a processing gas that contains a high concentration of methane ($CH_4$), ethylene ($C_2H_4$), and/or ethane ($C_2H_6$). Moreover, it is also possible to simultaneously measure the high concentrations of ethylene ($C_2H_4$) and ethane ($C_2H_6$).

In a case in which, using a multiple reflection cell or the like, the analysis device of the present invention measures a concentration of water ($H_2O$) at a low concentration of 100 ppm or less, the analysis device of the present invention calculates this concentration based on a water ($H_2O$) absorption between 5.89 ~ 6.12 $\mu$m. Here, the laser light source emits laser light having an oscillation wavelength that includes a wavelength between 5.89 - 6.12 $\mu$m.

A wavelength between 5.89 - 6.12 $\mu$m, or preferably a wavelength between 5.896 - 5.934 $\mu$m, or more preferably a wavelength of 5.8965 $\mu$m or 5.9353 $\mu$m is a wavelength in which one of the strongest absorption lines of water ($H_2O$) is present, and is where the absorption intensities of methane ($CH_4$), ethylene ($C_2H_4$), and/or ethane ($C_2H_6$), which are interference components in the processing gases of this wavelength region, are small, so that any interference effects from these are small. As a result, it is possible to improve the concentration measurement accuracy when measuring a low concentration of water ($H_2O$) in a processing gas that contains a high concentration of methane ($CH_4$), ethylene ($C_2H_4$), and/or ethane ($C_2H_6$).

A wavelength between 5.89 - 6.12 $\mu$m, or preferably a wavelength between 6.046 - 6.114 $\mu$m, or more preferably a wavelength of 6.0486 $\mu$m or 6.1138 $\mu$m is a wavelength in which one of the next strongest absorption lines of water ($H_2O$), after that in the above-described wavelength, is present, and is where the absorption intensities of methane ($CH_4$), ethylene ($C_2H_4$), and/or ethane ($C_2H_6$), which are interference components in the processing gases of this wavelength region, are small, so that any interference effects from these are small. As a result, it is possible to improve the concentration measurement accuracy when measuring a low concentration of water ($H_2O$) in a processing gas that contains a high concentration of methane ($CH_4$), ethylene ($C_2H_4$), and/or ethane ($C_2H_6$).

In a case in which, using a multiple reflection cell or the like, the analysis device of the present invention measures a concentration of acetylene ($C_2H_2$) at a low concentration of 100 ppm or less, the analysis device of the present invention calculates this concentration based on an acetylene ($C_2H_2$) absorption between 7.56 ~ 7.66 $\mu$m, or between 7.27 ~ 7.81 $\mu$m. Here, the laser light source emits laser light having an oscillation wavelength that includes a wavelength between 7.56 - 7.66 $\mu$m, or between 7.27 - 7.81 $\mu$m .

The strongest absorption line of acetylene ($C_2H_2$) is present in a wavelength band of 3.0 - 3.1 $\mu$m, however, it is difficult to produce this wavelength band using a quantum cascade laser. Note that a wavelength band of 3.0 - 3.1 $\mu$m is able to be produced using an interband cascade laser (ICL). In contrast, a wavelength between 7.56 - 7.66 $\mu$m, or preferably a wavelength between 7.594 - 7.651 $\mu$m is able to be produced using a quantum cascade laser, and is a wavelength in which the next strongest absorption line after that in the 3.0 - 3.1 $\mu$m wavelength band is present. More preferably, wavelengths of 7.5966 $\mu$m, 7.6233 $\mu$m, or 7.6501 $\mu$m are wavelengths in which the strongest absorption lines in this wavelength band are present, and are where the absorption intensities of methane ($CH_4$), ethylene ($C_2H_4$), and/or ethane ($C_2H_6$), which are interference components in the processing gases, are comparatively small, so that any interference effects from these are small. As a result, it is possible to improve the concentration measurement accuracy when measuring a low concentration of acetylene ($C_2H_2$) in a processing gas that contains a high concentration of methane ($CH_4$), ethylene ($C_2H_4$), and/or ethane ($C_2H_6$).

A wavelength between 7.56 - 7.66 $\mu$m, or preferably a wavelength between 7.566 - 7.634 $\mu$m, or more preferably a wavelength of 7.5698 $\mu$m, 7.6231 $\mu$m, or 7.6367 $\mu$m is a wavelength in which the absorption intensity is smaller than in the aforementioned wavelengths of 7.5966 $\mu$m, 7.6233 $\mu$m, or 7.6501 $\mu$m, however, the absorption intensities of methane ($CH_4$), ethylene ($C_2H_4$), and/or ethane ($C_2H_6$) are even smaller, so that any interference effects from these are also smaller. As a result, it is possible to improve the concentration measurement accuracy when measuring a low concentration of acetylene ($C_2H_2$) in a processing gas that contains a high concentration of methane ($CH_4$), ethylene ($C_2H_4$), and/or ethane ($C_2H_6$).

Moreover, in order to simultaneously measure a low concentration of 100 ppm or less of acetylene ($C_2H_2$) and an intermediate concentration of 1000 ppm or less of methane ($CH_4$), these concentrations are calculated based on an

acetylene ($C_2H_2$) absorption between 7.27 - 7.59 $\mu$m or between 7.64 - 7.81 $\mu$m. Here, the laser light source emits laser light having an oscillation wavelength that includes a wavelength between 7.27 - 7.59 $\mu$m, or between 7.64 - 7.81 $\mu$m. In this case, it is desirable that the methane concentrations be calculated based on an acetylene absorption between 7.378 - 7.638 $\mu$m, between 7.378 - 7.603 $\mu$m, or between 7.629 - 7.683 $\mu$m, and it is even more desirable that the methane concentrations be calculated based on an acetylene absorption at a wavelength of 7.5966 $\mu$m, 7.6501 $\mu$m, 7.5698 $\mu$m, or 7.6367 $\mu$m.

In a case in which, using a multiple reflection cell or the like, the analysis device of the present invention measures a concentration of methane ($CH_4$) at a low concentration of 100 ppm or less, the analysis device of the present invention calculates this concentration based on a methane ($CH_4$) absorption between 7.67 - 7.80 $\mu$m. Here, the laser light source emits laser light having an oscillation wavelength that includes a wavelength between 7.67 - 7.80 $\mu$m.

A wavelength between 7.67 - 7.80 $\mu$m, or preferably a wavelength between 7.670 - 7.792 $\mu$m, or more preferably a wavelength of 7.6704 $\mu$m or 7.7914 $\mu$m is a wavelength in which one of the strongest absorption lines of methane ($CH_4$) is present, and is where the absorption intensities of ethylene ($C_2H_4$) and/or ethane ($C_2H_6$), which are interference components in the processing gases of this wavelength region, are small, so that any interference effects from these are small. As a result, it is possible to improve the concentration measurement accuracy when measuring a low concentration of methane ($CH_4$) in a processing gas that contains a high concentration of ethylene ($C_2H_4$) and/or ethane ($C_2H_6$).

In a case in which, using a multiple reflection cell or the like, the analysis device of the present invention measures a concentration of methane ($CH_4$) at an intermediate concentration of from 100 ppm to 1%, the analysis device of the present invention calculates this concentration based on a methane ($CH_4$) absorption between 8.10 ~ 8.14 $\mu$m. Here, the laser light source emits laser light having an oscillation wavelength that includes a wavelength between 8.10 - 8.14 $\mu$m.

A wavelength between 8.10 - 8.14 $\mu$m, or preferably a wavelength between 8.107 - 8.139 $\mu$m, or more preferably a wavelength of 8.1073 $\mu$m or 8.1381 $\mu$m is a wavelength in which one of the intermediate strength absorption lines of methane ($CH_4$) is present, and is where the absorption intensities of ethylene ($C_2H_4$) and/or ethane ($C_2H_6$), which are interference components in the processing gases of this wavelength region, are small, so that any interference effects from these are small. As a result, it is possible to improve the concentration measurement accuracy when measuring an intermediate concentration of methane ($CH_4$) in a processing gas that contains a high concentration of ethylene ($C_2H_4$) and/or ethane ($C_2H_6$).

In a case in which, using a multiple reflection cell or the like, the analysis device of the present invention measures a concentration of methane ($CH_4$) at a high concentration of 1% or more, the analysis device of the present invention calculates this concentration based on a methane ($CH_4$) absorption between 8.10 - 8.13 $\mu$m. Here, the laser light source emits laser light having an oscillation wavelength that includes a wavelength between 8.10 - 8.13 $\mu$m.

A wavelength between 8.10 - 8.13 $\mu$m, or preferably a wavelength between 8.102 - 8.121 $\mu$m, or more preferably a wavelength of 8.1022 $\mu$m or 8.1206 $\mu$m is a wavelength in which one of the comparatively weak absorption lines of methane ($CH_4$) is present, and is where the absorption intensities of ethylene ($C_2H_4$) and/or ethane ($C_2H_6$), which are interference components in the processing gases of this wavelength region, are small, so that any interference effects from these are small. As a result, it is possible to improve the concentration measurement accuracy when measuring a high concentration of methane ($CH_4$) in a processing gas that contains a high concentration of ethylene ($C_2H_4$) or ethane ($C_2H_6$).

In a case in which, using a multiple reflection cell or the like, the analysis device of the present invention measures a concentration of ethylene ($C_2H_4$) at a high concentration of 1% or more, the analysis device of the present invention calculates this concentration based on an ethylene ($C_2H_4$) absorption between 8.46 ~ 8.60 $\mu$m. Here, the laser light source emits laser light having an oscillation wavelength that includes a wavelength between 8.46 - 8.60 $\mu$m.

A wavelength between 8.46 - 8.60 $\mu$m, or preferably a wavelength between 8.464 - 8.599 $\mu$m, or more preferably a wavelength of 8.4647 $\mu$m or 8.5981 $\mu$m is a wavelength in which one of the comparatively weak absorption lines of ethylene ($C_2H_4$) is present, and is where the absorption intensities of methane ($CH_4$) and/or ethane ($C_2H_6$), which are interference components in the processing gases of this wavelength region, are small, so that any interference effects from these are small. As a result, it is possible to improve the concentration measurement accuracy when measuring a high concentration of ethylene ($C_2H_4$) in a processing gas that contains a high concentration of methane ($CH_4$) and/or ethane ($C_2H_6$).

In a case in which, using a multiple reflection cell or the like, the analysis device of the present invention measures a concentration of ethane ($C_2H_6$) at a high concentration of 1% or more, the analysis device of the present invention calculates this concentration based on an ethane ($C_2H_6$) absorption between 6.13 ~ 6.14 $\mu$m or between 6.09 ~ 6.45 $\mu$m. Here, the laser light source emits laser light having an oscillation wavelength that includes a wavelength between 6.13 - 6.14 $\mu$m or between 6.09 - 6.45 $\mu$m. Note that in a case in which the analysis device of the present invention measures a high concentration of between no less than 1% and no more than 3% of ethane ($C_2H_6$), it is desirable that this concentration be calculated based on an ethane ($C_2H_6$) absorption between 6.09 - 6.45 $\mu$m.

A wavelength between 6.13 - 6.14 $\mu$m or between 6.09 - 6.45 $\mu$m, or preferably a wavelength between 6.135 - 6.139 $\mu$m or between 6.463 - 6.619 $\mu$m, or more preferably a wavelength of 6.1384 $\mu$m, 6.4673 $\mu$m, 6.5008 $\mu$m, 6.5624 $\mu$m, or 6.6145 $\mu$m is a wavelength in which one of the comparatively weak absorption lines of ethane ($C_2H_6$) is present, and is where the absorption intensities of methane ($CH_4$) and/or ethylene ($C_2H_4$), which are interference components in the processing gases of this wavelength region, are small, so that any interference effects from these are small. As a result, it is possible to improve the concentration measurement accuracy when measuring a high concentration of ethane ($C_2H_6$) in a processing gas that contains a high concentration of methane ($CH_4$) and/or ethylene ($C_2H_4$).

In a case in which, using a multiple reflection cell or the like, the analysis device of the present invention measures a concentration of ammonia ($NH_3$) at either a medium concentration of 100 ppm ~ 200 ppm, or a low concentration of 100 ppm or less, the analysis device of the present invention calculates this concentration based on an ammonia ($NH_3$) absorption between 6.06 - 6.25 $\mu$m or between 8.62 - 9.09 $\mu$m. Here, the laser light source emits laser light having an oscillation wavelength that includes a wavelength between 6.06 - 6.25 $\mu$m or between 8.62 - 9.09 $\mu$m. It is preferable that the concentrations be calculated based on an ammonia absorption between 6.141 - 6.153 $\mu$m or between 8.939 - 8.968 $\mu$m, and it is more preferable that the concentrations be calculated based on an ammonia absorption of 6.1450 $\mu$m, 6.1487 $\mu$m, 6.1496 $\mu$m, 8.9604 $\mu$m, 8.9473 $\mu$m, or 8.7671 $\mu$m .

In a case in which, using a multiple reflection cell or the like, the analysis device of the present invention measures a concentration of methanol ($CH_3OH$) at a high concentration of 1% or less, the analysis device of the present invention calculates this concentration based on a methanol absorption between 9.35 ~ 9.62 $\mu$m. Here, the laser light source emits laser light having an oscillation wavelength that includes a wavelength between 9.35 - 9.62 $\mu$m. It is preferable that the concentrations be calculated based on a methanol absorption between 9.477 - 9.526 $\mu$m, and it is more preferable that the concentrations be calculated based on a methanol absorption of 9.5168 $\mu$m, 9.5042 $\mu$m, or 9.4861 $\mu$m .

Furthermore, an analysis method according to the present invention is an analysis method in which a concentration of a measurement target component in the form of at least one of carbon dioxide, carbon monoxide, ethylene, ethane, water, acetylene, methane, ammonia, or methanol that are present in a processing gas is measured, and is characterized in that, in a case in which a concentration of the carbon dioxide is being measured, this concentration is calculated based on a carbon dioxide absorption between 4.23 ~ 4.24 $\mu$m, or 4.34 ~ 4.35 $\mu$m, in a case in which a concentration of the carbon monoxide is being measured, this concentration is calculated based on a carbon monoxide absorption between 4.59 ~ 4.61 $\mu$m, in a case in which a concentration of the water is being measured, this concentration is calculated based on a water absorption between 5.89 ~ 6.12 $\mu$m, in a case in which a concentration of the acetylene is being measured, this concentration is calculated based on an acetylene absorption between 7.56 - 7.66 $\mu$m, or 7.27 - 7.81 $\mu$m, in a case in which a concentration of the methane is being measured, this concentration is calculated based on a methane absorption between 7.67 - 7.80 $\mu$m, or 8.10 - 8.14 $\mu$m, in a case in which a concentration of the ethylene is being measured, this concentration is calculated based on an ethylene absorption between 8.46 - 8.60 $\mu$m, in a case in which a concentration of the ethane is being measured, this concentration is calculated based on an ethane absorption between 6.13 - 6.14 $\mu$m, or 6.09 - 6.45 $\mu$m, in a case in which a concentration of the ammonia is being measured, this concentration is calculated based on an ammonia absorption between 6.06 - 6.25 $\mu$m, or 8.62 - 9.09 $\mu$m, and in a case in which a concentration of the methanol is being measured, this concentration is calculated based on a methanol absorption between 9.35 - 9.62 $\mu$m.

[Effects of the Invention]

According to the above-described present invention, in an analysis device that utilizes light absorption, it is possible to reduce changes in an oscillation wavelength of a laser light source that are caused by fluctuations in the ambient temperature without having to use a reference cell into which a reference gas has been injected, and to thereby enable a concentration of a measurement target component to be measured accurately.

[Brief Description of the Drawings]

[FIG. 1] FIG. 1 is an overall schematic view of an analysis device according to an embodiment of the present invention.
[FIG. 2] FIG. 2 is a function block diagram of a signal processing device of the same embodiment.
[FIG. 3] FIG. 3 is a view showing a drive current (or voltage) and a modulation signal in a pseudo-continuous wave.
[FIG. 4] FIG. 4 is a schematic view showing a method of modulating a laser oscillation wavelength in the same embodiment.
[FIG. 5] FIG. 5 is a time series graph showing examples of an oscillation wavelength, a light intensity I (t), a logarithmic intensity L (t), a characteristic signal Fi (t), and a correlation value Si (t) in the same embodiment.

[FIG. 6] FIG. 6 is a view showing a wavelength shift and a modulation width shift in an intensity related signal (i.e., an absorption signal).

[FIG. 7] FIG. 7 is a graph showing (a) wavelength correction relational data, and (b) modulation correction relational data in the same embodiment.

[FIG. 8] FIG. 8 is a lookup table showing (a) wavelength correction relational data, and (b) modulation correction relational data in the same embodiment.

[FIG. 9] FIG. 9 shows conceptual views of concentration calculations made using independent correlation values and actual measurement correlation values of the same embodiment.

[FIG. 10] FIG. 10 is a function block diagram of a signal processing device of a variant embodiment.

[FIG. 11] FIG. 11 is an overall schematic view of an analysis device according to a variant embodiment.

[FIG. 12] FIG. 12 is a schematic view showing spectrum changes caused by coexistence effects and spectrum changes caused by pressure fluctuations.

[Best Embodiments for Implementing the Invention]

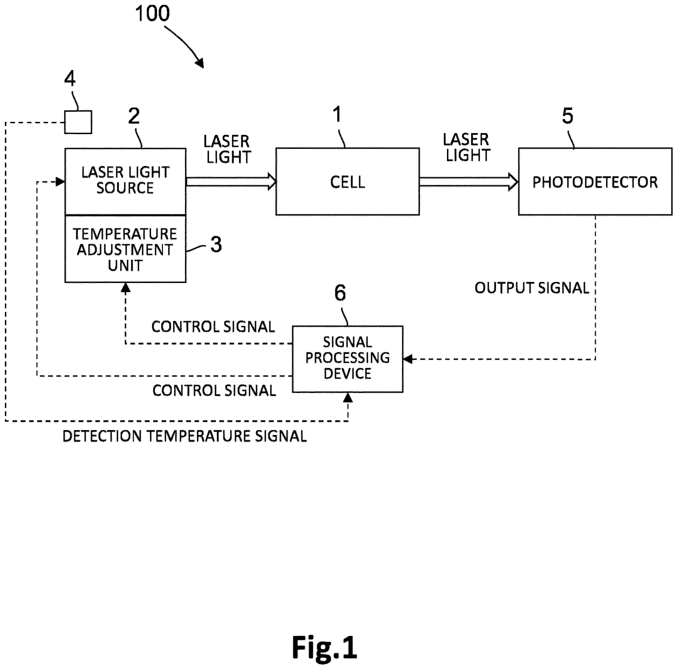

An analysis device 100 of the present embodiment is a concentration measurement device that measures a concentration of a measurement target component that is contained in a sample gas such as a combustion gas such as a gas currently being combusted or a combustion exhaust gas or the like, or a processing gas or the like. As is shown in FIG. 1, the analysis device 100 of the present embodiment is provided with a cell 1 into which a sample gas is introduced, a semiconductor laser 2 that serves as a laser light source for irradiating onto the cell 1 laser light that is to be modulated, a temperature adjustment unit 3 that adjusts a temperature of the semiconductor laser 2, a temperature sensor 4 that detects an ambient temperature around the semiconductor laser 2, a photodetector 5 that is provided on an optical path of sample light which is the laser light transmitted through the cell 1 and that optically receives the sample light, and a signal processing device 6 that receives an output signal from the photodetector 5 and calculates a concentration of a measurement target component based on a value of this output signal. Here, a 'gas currently being combusted' refers to a gas being combusted in an internal combustion engine of an automobile or the like, an external combustion engine, an industrial furnace, an incinerator, a turbine, or a power plant or the like. A 'combustion exhaust gas' refers to a gas that, having already been combusted, is then expelled from an internal combustion engine of an automobile or the like, an external combustion engine, an industrial furnace, an incinerator, a turbine, or a power plant or the like. Moreover, a 'processing gas' refers to a gas used in a chemical plant such as a petrochemical plant, a coal chemical plant, a natural gas chemical plant, an oil refinery plant, a methanation plant, or a gasification furnace or the like. In addition to raw material gases such as natural gas and the like, 'processing gas' may include gases separated in a chemical plant or gases created in a chemical plant.

Note that an introduction flow path that is used to introduce a sampling gas into the analysis device 100, and a discharge flow path through which gas that has been analyzed by the analysis device 100 is discharged are connected to the analysis device 100 of the present embodiment. In addition, a pump that is used to introduce a sampling gas to the analysis device 100 is provided on the introduction flow path or the discharge flow path. Moreover, the introduction flow path may be formed in such a way that exhaust gas is sampled directly from an exhaust pipe or the like, or in such a way that exhaust gas is introduced from a bag in which the exhaust gas has first been collected, or in such a way that exhaust gas that has been diluted by a dilution device such as, for example, a CVS (Constant Volume Sampler) or the like is introduced.

Each of the aforementioned portions will now be described.

The cell 1 is formed from a transparent substance such as quartz, calcium fluoride, or barium fluoride or the like that have substantially no light absorption in an absorption wavelength region of the measurement target component, and includes a light entry port and a light exit port. An inlet port (not shown in the drawings) that is used to introduce gas into an interior thereof, and an outlet port (not shown in the drawings) that is used to discharge gas from the interior thereof are provided in the cell 1, and a sample gas is introduced into the cell 1 through this inlet port.

The semiconductor laser 2 used here is a quantum cascade laser (QCL), which is one type of semiconductor laser 2, that oscillates mid-infrared laser light (4~12 $\mu$m). This semiconductor laser 2 is able to modulate (i.e., alter) an oscillation wavelength by means of an applied current (or voltage). Note that as long as the oscillation wavelength is able to be varied, then it is also possible for another type of laser to be used, or for a temperature or the like to be altered in order to change the oscillation wavelength.

The temperature adjustment unit 3 adjusts a temperature of the semiconductor laser 2 and employs a thermoelectric conversion element such as, for example, a Peltier element or the like. An upper surface of the temperature adjustment unit 3 of the present embodiment is formed as a heat absorption surface on which are mounted the semiconductor laser 2 and a temperature sensor (not shown in the drawings) that is used to detect the temperature of the semiconductor laser 2, while a lower surface thereof is formed as a heat dissipation surface in which is provided a heat sink (not shown in the drawings) such as, for example, heat dissipation fins or the like. The temperature adjustment unit 3 is able to adjust the temperature of the semiconductor laser 2 as a result of a DC voltage (or DC current) that is applied thereto being controlled in accordance with a target temperature supplied by a temperature control unit 72 (described below).

The temperature sensor 4 detects an ambient temperature around the semiconductor laser 2. Here, the temperature sensor 4 either detects a temperature of an atmosphere inside a package in which the semiconductor laser and the temperature adjustment unit 3 are housed, or detects an ambient temperature in the vicinity of an exterior of this package.

The photodetector 5 in this case is formed by a thermal type of detector such as a comparatively low-cost thermopile or the like, however, other types of detectors such as, for example, quantum photoelectric elements such as HgCdTe, InGaAs, InAsSb, or PbSe devices or the like which have high responsivity may also be used.

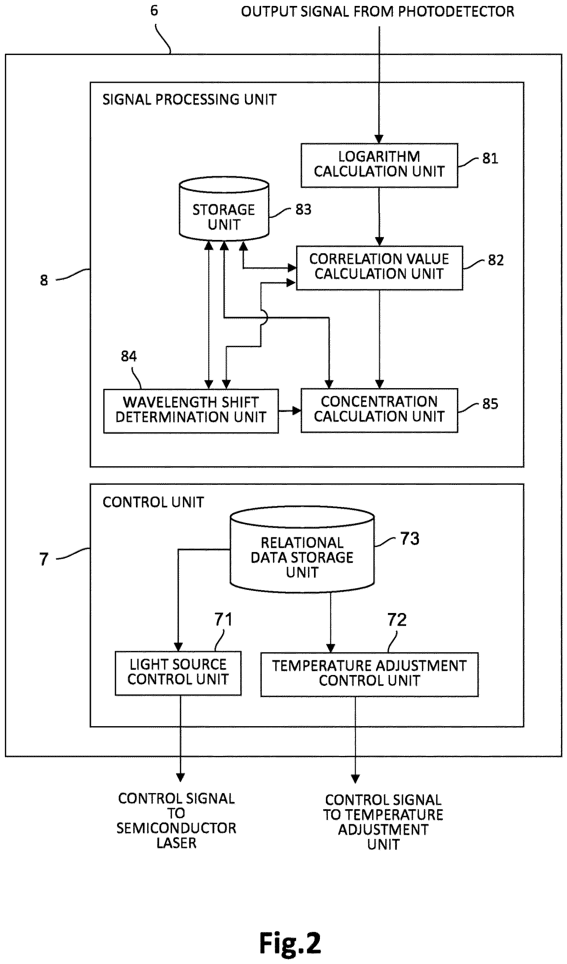

The signal processing device 6 is equipped with analog electrical circuits formed by buffers and amplifiers and the like, digital electrical circuits formed by a CPU and memory and the like, and at least one of an AD converter or DA converter that interfaces between these analog or digital electrical circuits. As a result of the CPU and peripheral devices thereof operating in mutual collaboration in accordance with a predetermined program stored in a predetermined area of the memory, as is shown in FIG. 2, the signal processing device 6 functions as a control unit 7 that controls the semiconductor laser 2 and the temperature adjustment unit 3, and as a signal processing unit 8 that receives output signals from the photodetector 5 and then performs arithmetic processing on the values contained therein so as to calculate the concentration of a measurement target component.

Each of these portions will now be described in detail.

The control unit 7 is provided with a light source control unit 71 that controls the oscillation and the modulation width of the semiconductor laser 2, and a temperature control unit 72 that controls the temperature adjustment unit 3 so that a predetermined temperature is achieved.

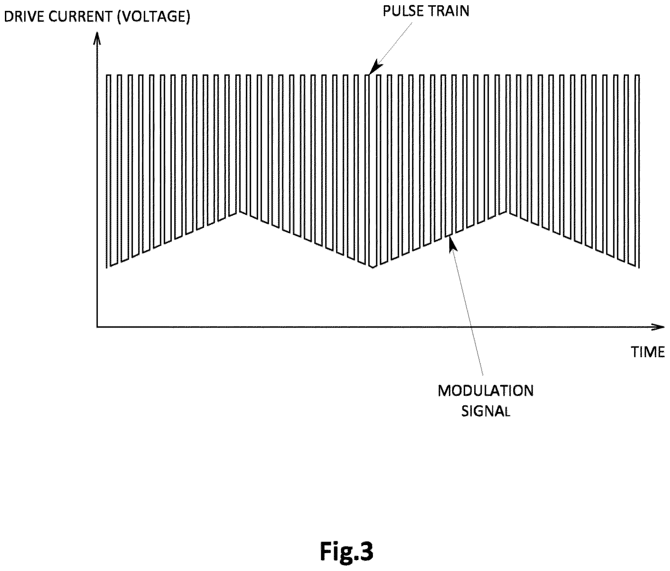

The light source control unit 71 outputs a current (or a voltage) control signal so as to control a current source (or a voltage source) that drives the semiconductor laser 2. More specifically, as is shown in FIG. 3, separately from the drive current (or drive voltage) that causes the semiconductor laser 2 to perform a pulse oscillation, the light source control unit 71 causes a drive current (or drive voltage) that imparts wavelength modulation to be changed at a predetermined frequency, and causes the oscillation wavelength of the laser light output from the semiconductor laser 2 to be modulated at a predetermined frequency relative to a central wavelength. As a result, the semiconductor laser 2 is able to emit modulation light that has been modulated by a predetermined modulation frequency.

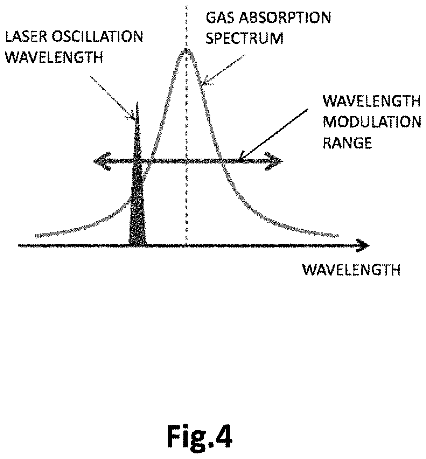

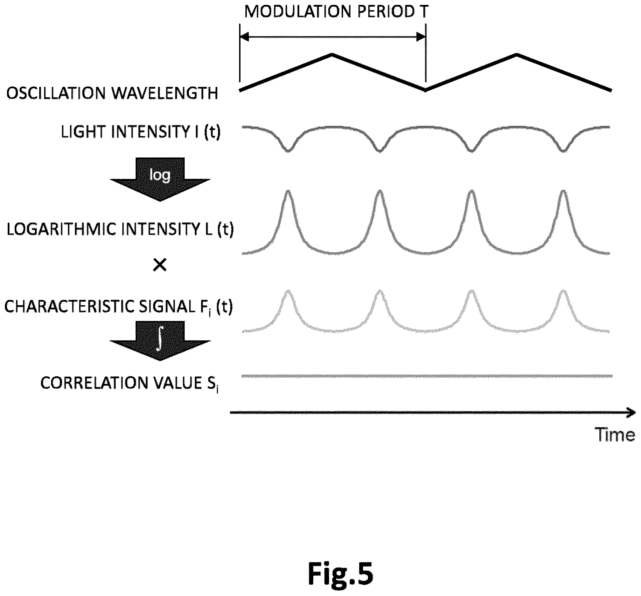

In this embodiment, the light source control unit 71 changes the drive current into a triangular wave configuration so as to modulate the oscillation wavelength into a triangular wave configuration (see 'oscillation wavelength' in FIG. 5). In actuality, the modulation of the drive current is performed using a separate function in order for the oscillation wavelength to have a triangular configuration. Moreover, as is shown in FIG. 4, the oscillation wavelength of the laser light is modulated such that a peak of the light absorption spectrum of the measurement target component forms a central wavelength thereof. In addition, it is also possible for the light source control unit 71 to change the drive current into a sinusoidal wave shape or a sawtooth wave shape, or into a desired function shape, and to modulate the oscillation wavelength into a sinusoidal wave shape or a sawtooth wave shape, or into a desired function shape.

More specifically, in a case in which the analysis device 100 measures a concentration of at least one of nitric oxide (NO), nitrogen dioxide (NOz), nitrous oxide ($N_2O$), ammonia ($NH_3$), ethane ($C_2H_6$), formaldehyde (HCHO), acetaldehyde ($CH_3CHO$), sulfur dioxide (SOz), methane ($CH_4$), methanol ($CH_3OH$), or ethanol ($C_2H_5OH$) that are present in a combustion gas, the light source control unit 71 controls the modulation of the semiconductor laser 2 so that the wavelength modulation ranges given below are achieved. Note that a suitable semiconductor laser that is able to emit modulation light modulated in the wavelength modulation ranges given below is selected as the semiconductor laser 2.

In a case in which the measurement target component is nitric oxide (NO) at a low concentration of 100 ppm or less, the light source control unit 71 controls the modulation of the semiconductor laser 2 such that the wavelength modulation range of the laser light includes a wavelength between 5.24 ~ 5.26 $\mu$m. More specifically, the light source control unit 71 controls the modulation of the semiconductor laser 2 such that the wavelength modulation range of the laser light preferably includes a wavelength between 5.245 - 5.247 $\mu$m, or more preferably a wavelength of 5.2462 $\mu$m. By modulating the wavelength modulation range in this way, it is possible to reduce any interference effects from water ($H_2O$), carbon dioxide (COz), and/or ethylene ($C_2H_4$), and to thereby improve the concentration measurement accuracy when measuring a low concentration of nitric oxide (NO).

In a case in which the measurement target component is nitrogen dioxide (NOz) at a low concentration of 100 ppm or less, the light source control unit 71 controls the modulation of the semiconductor laser 2 such that the wavelength modulation range of the laser light includes a wavelength between 6.14 ~ 6.26 $\mu$m. More specifically, the light source control unit 71 controls the modulation of the semiconductor laser 2 such that the wavelength modulation range of the laser light preferably includes a wavelength between 6.145 - 6.254 $\mu$m, or more preferably a wavelength of 6.2322 $\mu$m or 6.2538 $\mu$m. By modulating the wavelength modulation range in this way, it is possible to reduce any interference effects from water ($H_2O$) and/or ammonia ($NH_3$), and to thereby improve the concentration measurement accuracy when measuring a low concentration of nitrogen dioxide (NOz).

In a case in which the measurement target component is a low concentration of 100 ppm or less of nitrous oxide ($N_2O$), the light source control unit 71 controls the modulation of the semiconductor laser 2 such that the wavelength modulation range of the laser light includes a wavelength between 7.84 ~ 7.91 $\mu$m. More specifically, the light source control unit 71 controls the modulation of the semiconductor laser 2 such that the wavelength modulation range of the laser light preferably includes a wavelength between 7.845 - 7.907 $\mu$m, or more preferably a wavelength of 7.8455 $\mu$m, 7.8509 $\mu$m, 7.8784 $\mu$m, or 7.9067 $\mu$m. By modulating the wavelength modulation range in this way, it is possible to reduce any interference effects from water ($H_2O$), methane ($CH_4$), and/or acetylene ($C_2H_2$), and to thereby improve the concentration measurement accuracy when measuring a low concentration of nitrous oxide ($N_2O$).

In a case in which the measurement target component is ammonia ($NH_3$) at a low concentration of 100 ppm or less, the light source control unit 71 controls the modulation of the semiconductor laser 2 such that the wavelength modulation range of the laser light includes a wavelength between 9.38 ~ 9.56 $\mu$m. More specifically, the light source control unit 71 controls the modulation of the semiconductor laser 2 such that the wavelength modulation range of the laser light preferably includes a wavelength between 9.384 - 9.557 $\mu$m, or more preferably a wavelength of 9.3847 $\mu$m or 9.5566 $\mu$m. By modulating the wavelength modulation range in this way, it is possible to reduce any interference effects from water ($H_2O$), carbon dioxide ($COz$), and/or ethylene ($C_2H_4$), and to thereby improve the concentration measurement accuracy when measuring a low concentration of ammonia ($NH_3$).

In a case in which the measurement target component is ethane ($C_2H_6$) at a low concentration of 100 ppm or less, the light source control unit 71 controls the modulation of the semiconductor laser 2 such that the wavelength modulation range of the laser light includes a wavelength between 3.33 - 3.36 $\mu$m. More specifically, the light source control unit 71 controls the modulation of the semiconductor laser 2 such that the wavelength modulation range of the laser light preferably includes a wavelength between 3.336 - 3.352 $\mu$m, or more preferably a wavelength of 3.3368 $\mu$m, 3.3482 $\mu$m or 3.3519 $\mu$m. By modulating the wavelength modulation range in this way, it is possible to reduce any interference effects from water ($H_2O$), methane ($CH_4$), and/or ethylene ($C_2H_4$), and to thereby improve the concentration measurement accuracy when measuring a low concentration of ethane ($C_2H_6$).

In a case in which the measurement target component is formaldehyde (HCHO) at a low concentration of 100 ppm or less, the light source control unit 71 controls the modulation of the semiconductor laser 2 such that the wavelength modulation range of the laser light includes a wavelength between 5.65 ~ 5.67 $\mu$m. More specifically, the light source control unit 71 controls the modulation of the semiconductor laser 2 such that the wavelength modulation range of the laser light preferably includes a wavelength between 5.651 - 5.652 $\mu$m, or more preferably a wavelength of 5.6514 $\mu$m. By modulating the wavelength modulation range in this way, it is possible to reduce any interference effects from water ($H_2O$) and/or ammonia ($NH_3$), and to thereby improve the concentration measurement accuracy when measuring a low concentration of formaldehyde (HCHO). Moreover, because such a wavelength coincides with a strong absorption band of acetaldehyde ($CH_3CHO$), it is possible to simultaneously measure the formaldehyde (HCHO) and the acetaldehyde ($CH_3CHO$).

The light source control unit 71 is also able to control the modulation of the semiconductor laser 2 such that the wavelength modulation range of the laser light preferably includes a wavelength between 5.665 - 5.667 $\mu$m, or more preferably a wavelength of 5.6660 $\mu$m. The absorption intensity of formaldehyde (HCHO) is somewhat smaller than in the aforementioned wavelength of 5.6514 $\mu$m, however, the absorption intensity of water ($H_2O$) is even less, so that any interference effects therefrom are even smaller. As a result, it is possible to improve the measurement accuracy when measuring a concentration of formaldehyde (HCHO). Moreover, because such a wavelength coincides with a strong absorption band of acetaldehyde ($CH_3CHO$), it is possible to measure the acetaldehyde ($CH_3CHO$), or to simultaneously measure the formaldehyde (HCHO) and the acetaldehyde ($CH_3CHO$).

In a case in which the measurement target component is sulfur dioxide (SOz) at a low concentration of 100 ppm or less, the light source control unit 71 controls the modulation of the semiconductor laser 2 such that the wavelength modulation range of the laser light includes a wavelength between 7.38 ~ 7.42 $\mu$m. More specifically, the light source control unit 71 controls the modulation of the semiconductor laser 2 such that the wavelength modulation range of the laser light preferably includes a wavelength between 7.385 - 7.417 $\mu$m, or more preferably a wavelength of 7.3856 $\mu$m or 7.4163 $\mu$m. By modulating the wavelength modulation range in this way, it is possible to reduce any interference effects from water ($H_2O$), methane ($CH_4$), acetylene ($C_2H_2$), and/or nitrous oxide ($N_2O$), and to thereby improve the concentration measurement accuracy when measuring a low concentration of sulfur dioxide (SOz).

In a case in which the measurement target component is methane ($CH_4$) at a low concentration of 100 ppm or less, the light source control unit 71 controls the modulation of the semiconductor laser 2 such that the wavelength modulation range of the laser light includes a wavelength between 7.50 - 7.54 $\mu$m. More specifically, the light source control unit 71 controls the modulation of the semiconductor laser 2 such that the wavelength modulation range of the laser light preferably includes a wavelength between 7.503 - 7.504 $\mu$m, or more preferably a wavelength of 7.5035 $\mu$m. By modulating the wavelength modulation range in this way, it is possible to reduce any interference effects from water ($H_2O$), sulfur dioxide (SOz), acetylene ($C_2H_2$), and/or nitrous oxide ($N_2O$), and to thereby improve the concentration measurement accuracy when measuring a low concentration of methane ($CH_4$). Moreover, as a result of the wavelength range being modulated so as to include a wavelength of 7.5035 $\mu$m, there is a water ($H_2O$) absorption line in the vicinity of this wavelength so that it is possible to simultaneously measure methane ($CH_4$) and water ($H_2O$).

The light source control unit 71 is also able to control the modulation of the semiconductor laser 2 such that the wavelength modulation range of the laser light preferably includes a wavelength between 7.535 - 7.536 $\mu$m, or more preferably a wavelength of 7.5354 $\mu$m. This wavelength has substantially the same methane ($CH_4$) absorption intensity as the aforementioned 7.5035 $\mu$m, and weaker absorption intensities of water ($H_2O$), sulfur dioxide ($SO_2$), acetylene ($C_2H_2$), and/or nitrous oxide ($N_2O$), which are interference components in the combustion gases of this wavelength region, so that any interference effects from these are smaller. As a result, it is possible to improve the measurement accuracy when measuring a concentration of methane ($CH_4$).

In a case in which the measurement target component is methanol ($CH_3OH$) at a low concentration of 100 ppm or less, the light source control unit 71 controls the modulation of the semiconductor laser 2 such that the wavelength modulation range of the laser light includes a wavelength between 9.45 ~ 9.47 $\mu$m. More specifically, the light source control unit 71 controls the modulation of the semiconductor laser 2 such that the wavelength modulation range of the laser light preferably includes a wavelength between 9.467 - 9.468 $\mu$m, or more preferably a wavelength of 9.4671 $\mu$m. By modulating the wavelength modulation range in this way, it is possible to reduce any interference effects from ethylene ($C_2H_4$) ammonia ($NH_3$), and/or carbon dioxide ($CO_2$) and to thereby improve the concentration measurement accuracy when measuring a low concentration of methanol ($CH_3OH$). Moreover, because such a wavelength coincides with a strong absorption band of ethanol ($C_2H_5OH$), it is possible to simultaneously measure the methanol ($CH_3OH$) and the ethanol ($C_2H_5OH$).

The light source control unit 71 is also able to control the modulation of the semiconductor laser 2 such that the wavelength modulation range of the laser light preferably includes a wavelength between 9.455 - 9.456 $\mu$m, or more preferably a wavelength of 9.4557 $\mu$m. This wavelength has substantially the same methanol ($CH_3OH$) or ethanol ($C_2H_5OH$) absorption intensity as the aforementioned 9.4671 $\mu$m, and weaker absorption intensities of ethylene ($C_2H_4$), ammonia ($NH_3$), and/or carbon dioxide ($CO_2$), which are interference components in the combustion gases of this wavelength region, so that any interference effects from these are smaller. As a result, it is possible to improve the measurement accuracy when measuring a concentration of methanol ($CH_3OH$) or ethanol ($C_2H_5OH$). Moreover, it is possible to simultaneously measure the methanol ($CH_3OH$) and the ethanol ($C_2H_5OH$).

Moreover, in a case in which the analysis device 100 measures a concentration of at least one of carbon dioxide ($CO_2$), carbon monoxide (CO), ethylene ($C_2H_4$), ammonia ($NH_3$), ethane ($C_2H_6$), water ($H_2O$), acetylene ($C_2H_2$), methane ($CH_4$), or methanol ($CH_3OH$), that are present in a processing gas, the light source control unit 71 controls the modulation of the semiconductor laser 2 so that the wavelength modulation ranges given below are achieved.

In a case in which the measurement target component is carbon dioxide ($CO_2$) at a low concentration of 100 ppm or less, the light source control unit 71 controls the modulation of the semiconductor laser 2 such that the wavelength modulation range of the laser light includes a wavelength between 4.23 ~ 4.24 $\mu$m. More specifically, the light source control unit 71 controls the modulation of the semiconductor laser 2 such that the wavelength modulation range of the laser light preferably includes a wavelength between 4.234 - 4.238 $\mu$m or between 4.235 - 4.238 $\mu$m, or more preferably a wavelength of 4.2347 $\mu$m or 4.2371 $\mu$m. By modulating the wavelength modulation range in this way, it is possible to reduce any interference effects from methane ($CH_4$), ethylene ($C_2H_4$), and/or ethane ($C_2H_6$), and to thereby improve the concentration measurement accuracy when measuring a low concentration of carbon dioxide ($CO_2$) in a processing gas containing a high concentration of methane ($CH_4$), ethylene ($C_2H_4$), and/or ethane ($C_2H_6$).

In a case in which the measurement target component is carbon dioxide ($CO_2$) at an intermediate concentration of from 100 ppm to 1%, the light source control unit 71 controls the modulation of the semiconductor laser 2 such that the wavelength modulation range of the laser light includes a wavelength between 4.34 - 4.35 $\mu$m. More specifically, the light source control unit 71 controls the modulation of the semiconductor laser 2 such that the wavelength modulation range of the laser light preferably includes a wavelength between 4.342 - 4.347 $\mu$m, or more preferably a wavelength of 4.3428 $\mu$m or 4.3469 $\mu$m. By modulating the wavelength modulation range in this way, it is possible to reduce any interference effects from methane ($CH_4$), ethylene ($C_2H_4$), and/or ethane ($C_2H_6$), and to thereby improve the concentration measurement accuracy when measuring an intermediate concentration of carbon dioxide ($CO_2$) in a processing gas containing a high concentration of methane ($CH_4$), ethylene ($C_2H_4$), and/or ethane ($C_2H_6$).

In a case in which the measurement target component is carbon monoxide (CO) at a low concentration of 100 ppm or less, the light source control unit 71 controls the modulation of the semiconductor laser 2 such that the wavelength modulation range of the laser light includes a wavelength between 4.59 ~ 4.61 $\mu$m or between 4.59 ~ 4.60 $\mu$m. More specifically, the light source control unit 71 controls the modulation of the semiconductor laser 2 such that the wavelength modulation range of the laser light preferably includes a wavelength between 4.594 - 4.604 $\mu$m, or more preferably a wavelength of 4.5950 $\mu$m or 4.6024 $\mu$m. By modulating the wavelength modulation range in this way, it is possible to reduce any interference effects from methane ($CH_4$), ethylene ($C_2H_4$), and/or ethane ($C_2H_6$), and to thereby improve the concentration measurement accuracy when measuring a low concentration of carbon monoxide (CO) in a processing gas containing a high concentration of methane ($CH_4$), ethylene ($C_2H_4$), and/or ethane ($C_2H_6$).

In a case in which the measurement target component is water ($H_2O$) at a low concentration of 100 ppm or less, the light source control unit 71 controls the modulation of the semiconductor laser 2 such that the wavelength modulation range of the laser light includes a wavelength between 5.89 ~ 6.12 $\mu$m. More specifically, the light source control unit 71 controls the modulation of the semiconductor laser 2 such that the wavelength modulation range of the laser light preferably includes a wavelength between 5.896 ~ 5.934 $\mu$m, or more preferably a wavelength of 5.8965 $\mu$m or 5.9353 $\mu$m. By modulating the wavelength modulation range in this way, it is possible to reduce any interference effects from methane ($CH_4$), ethylene ($C_2H_4$), and/or ethane ($C_2H_6$), and to thereby improve the concentration measurement accuracy when measuring a low concentration of water ($H_2O$) in a processing gas containing a high concentration of methane ($CH_4$), ethylene ($C_2H_4$), and/or ethane ($C_2H_6$).

The light source control unit 71 is also able to control the modulation of the semiconductor laser 2 such that the wavelength modulation range of the laser light preferably includes a wavelength between 6.046 - 6.114 $\mu$m, or more preferably a wavelength of 6.0486 $\mu$m or 6.1138 $\mu$m. By modulating the wavelength modulation range in this way, it is possible to reduce any interference effects from methane ($CH_4$), ethylene ($C_2H_4$), and/or ethane ($C_2H_6$), and to thereby improve the concentration measurement accuracy when measuring a low concentration of water ($H_2O$) in a processing gas containing a high concentration of methane ($CH_4$), ethylene ($C_2H_4$), and/or ethane ($C_2H_6$).

In a case in which the measurement target component is acetylene ($C_2H_2$) at a low concentration of 100 ppm or less, the light source control unit 71 controls the modulation of the semiconductor laser 2 such that the wavelength modulation range of the laser light includes a wavelength between 7.56 - 7.66 $\mu$m, between 7.27 - 7.81 $\mu$m, between 7.27 - 7.24 $\mu$m, or between 7.25 - 7.81 $\mu$m. More specifically, the light source control unit 71 controls the modulation of the semiconductor laser 2 such that the wavelength modulation range of the laser light preferably includes a wavelength between 7.378 - 7.638 $\mu$m, between 7.378 - 7.603 $\mu$m, between 7.378 - 7.420 $\mu$m, between 7.430 - 7.603 $\mu$m, between 7.430 - 7.638 $\mu$m, between 7.629 - 7.683 $\mu$m, or between 7.594 - 7.651 $\mu$m, or more preferably a wavelength of 7.5966 $\mu$m, 7.6233 $\mu$m or 7.6501 $\mu$m. By modulating the wavelength modulation range in this way, it is possible to reduce any interference effects from methane ($CH_4$), ethylene ($C_2H_4$), and/or ethane ($C_2H_6$), and to thereby improve the concentration measurement accuracy when measuring a low concentration of acetylene ($C_2H_2$) in a processing gas containing a high concentration of methane ($CH_4$), ethylene ($C_2H_4$), and/or ethane ($C_2H_6$).

The light source control unit 71 is also able to control the modulation of the semiconductor laser 2 such that the wavelength modulation range of the laser light preferably includes a wavelength between 7.566 - 7.634 $\mu$m, or more preferably a wavelength of 7.5698 $\mu$m, 7.6231 $\mu$m, or 7.6367 $\mu$m. By modulating the wavelength modulation range in this way, it is possible to reduce any interference effects from methane ($CH_4$), ethylene ($C_2H_4$), and/or ethane ($C_2H_6$), and to thereby improve the concentration measurement accuracy when measuring a low concentration of acetylene ($C_2H_2$) in a processing gas containing a high concentration of methane ($CH_4$), ethylene ($C_2H_4$), and/or ethane ($C_2H_6$).

In a case in which the measurement target component is methane ($CH_4$) at a low concentration of 100 ppm or less, the light source control unit 71 controls the modulation of the semiconductor laser 2 such that the wavelength modulation range of the laser light includes a wavelength between 7.67 - 7.80 $\mu$m. More specifically, the light source control unit 71 controls the modulation of the semiconductor laser 2 such that the wavelength modulation range of the laser light preferably includes a wavelength between 7.670 - 7.792 $\mu$m, or more preferably a wavelength of 7.6704 $\mu$m or 7.7914 $\mu$m. By modulating the wavelength modulation range in this way, it is possible to reduce any interference effects from ethylene ($C_2H_4$) and/or ethane ($C_2H_6$), and to thereby improve the concentration measurement accuracy when measuring a low concentration of methane ($CH_4$) in a processing gas containing a high concentration of ethylene ($C_2H_4$) and/or ethane ($C_2H_6$).

In a case in which the measurement target component is methane ($CH_4$) at an intermediate concentration of from 100 ppm to 1%, the light source control unit 71 controls the modulation of the semiconductor laser 2 such that the wavelength modulation range of the laser light includes a wavelength between 8.10 - 8.14 $\mu$m. More specifically, the light source control unit 71 controls the modulation of the semiconductor laser 2 such that the wavelength modulation range of the laser light preferably includes a wavelength between 8.107 - 8.139 $\mu$m, or more preferably a wavelength of 8.1073 $\mu$m or 8.1381 $\mu$m. By modulating the wavelength modulation range in this way, it is possible to reduce any interference effects from ethylene ($C_2H_4$) and/or ethane ($C_2H_6$), and to thereby improve the concentration measurement accuracy when measuring an intermediate concentration of methane ($CH_4$) in a processing gas containing a high concentration of ethylene ($C_2H_4$) and/or ethane ($C_2H_6$).

In a case in which the measurement target component is methane ($CH_4$) at a high concentration of 1% or more, the light source control unit 71 controls the modulation of the semiconductor laser 2 such that the wavelength modulation range of the laser light includes a wavelength between 8.10 - 8.13 $\mu$m. More specifically, the light source control unit 71 controls the modulation of the semiconductor laser 2 such that the wavelength modulation range of the laser light preferably includes a wavelength between 8.102 - 8.121 $\mu$m, or more preferably a wavelength of 8.1022 $\mu$m or 8.1206 $\mu$m. By modulating the wavelength modulation range in this way, it is possible to reduce any interference effects from ethylene ($C_2H_4$) and/or ethane ($C_2H_6$), and to thereby improve the concentration measurement accuracy when measuring a high concentration of methane ($CH_4$) in a processing gas containing a high concentration of ethylene ($C_2H_4$) and/or ethane ($C_2H_6$).

In a case in which the measurement target component is methane ($CH_4$) at a high concentration of 1% or more, the light source control unit 71 controls the modulation of the semiconductor laser 2 such that the wavelength modulation range of the laser light includes a wavelength between 8.10 - 8.13 $\mu$m. More specifically, the light source control unit 71 controls the modulation of the semiconductor laser 2 such that the wavelength modulation range of the laser light includes a wavelength of 8.1022 $\mu$m or 8.1206 $\mu$m. By modulating the wavelength modulation range in this way, it is possible to reduce any interference effects from ethylene ($C_2H_4$) and/or ethane ($C_2H_6$), and to thereby improve the concentration measurement accuracy when measuring a high concentration of methane ($CH_4$) in a processing gas containing a high concentration of ethylene ($C_2H_4$) and/or ethane ($C_2H_6$).

In a case in which the measurement target component is ethylene ($C_2H_4$) at a high concentration of 1% or more, the light source control unit 71 controls the modulation of the semiconductor laser 2 such that the wavelength modulation range of the laser light includes a wavelength between 8.46 ~ 8.60 $\mu$m. More specifically, the light source control unit 71 controls the modulation of the semiconductor laser 2 such that the wavelength modulation range of the laser light preferably includes a wavelength between 8.464 - 8.599 $\mu$m, or more preferably a wavelength of 8.4647 $\mu$m or 8.5981 $\mu$m. By modulating the wavelength modulation range in this way, it is possible to reduce any interference effects from methane ($CH_4$) and/or ethane ($C_2H_6$), and to thereby improve the concentration measurement accuracy when measuring a high concentration of ethylene ($C_2H_4$) in a processing gas containing a high concentration of methane ($CH_4$) and/or ethane ($C_2H_6$).

In a case in which the measurement target component is ethane ($C_2H_6$) at a high concentration of 1% or more, the light source control unit 71 controls the modulation of the semiconductor laser 2 such that the wavelength modulation range of the laser light includes a wavelength between 6.13 ~ 6.14 $\mu$m, between 6.09 ~ 6.45 $\mu$m, between 6.09 ~ 6.39 $\mu$m, or between 6.41 - 6.45 $\mu$m. More specifically, the light source control unit 71 controls the modulation of the semiconductor laser 2 such that the wavelength modulation range of the laser light preferably includes a wavelength between 6.135 - 6.139 $\mu$m or between 6.463 - 6.619 $\mu$m, or more preferably a wavelength of 6.1384 $\mu$m, 6.4673 $\mu$m, 6.5008 $\mu$m, 6.5624 $\mu$m or 6.6145 $\mu$m. By modulating the wavelength modulation range in this way, it is possible to reduce any interference effects from methane ($CH_4$) and/or ethylene ($C_2H_4$), and to thereby improve the concentration measurement accuracy when measuring a high concentration of ethane ($C_2H_6$) in a processing gas containing a high concentration of methane ($CH_4$) and/or ethylene ($C_2H_4$).

In a case in which the measurement target component is ammonia ($NH_3$) at either an intermediate concentration of between 100 ppm ~ 200 ppm or at a low concentration of 100 ppm or less, the light source control unit 71 controls the modulation of the semiconductor laser 2 such that the wavelength modulation range of the laser light includes a wavelength between 6.06 - 6.25 $\mu$m, between 6.06 - 6.14 $\mu$m, between 6.15 - 6.17 $\mu$m, between 6.19 - 6.25 $\mu$m, or between 8.62 - 9.09 $\mu$m. More specifically, the light source control unit 71 controls the modulation of the semiconductor laser 2 such that the wavelength modulation range of the laser light preferably includes a wavelength between 6.141 - 6.153 $\mu$m, between 6.141 - 6.149 $\mu$m, between 6.150 - 6.153 $\mu$m or between 8.939 - 8.968 $\mu$m, or more preferably a wavelength of 6.1450 $\mu$m, 6.1487 $\mu$m, 6.1496 $\mu$m, 8.9604 $\mu$m, 8.9473 $\mu$m, or 8.7671 $\mu$m. By modulating the wavelength modulation range in this way, it is possible to reduce any interference effects from methane ($CH_4$) and/or ethylene ($C_2H_4$), and to thereby improve the concentration measurement accuracy when measuring an intermediate concentration or a low concentration of ammonia ($NH_3$) in a processing gas containing a high concentration of methane ($CH_4$) and/or ethylene ($C_2H_4$).

In a case in which the measurement target component is methanol ($CH_3OH$) at a high concentration of 1% or less, the light source control unit 71 controls the modulation of the semiconductor laser 2 such that the wavelength modulation range of the laser light includes a wavelength between 9.35 - 9.62 $\mu$m. More specifically, the light source control unit 71 controls the modulation of the semiconductor laser 2 such that the wavelength modulation range of the laser light preferably includes a wavelength between 9.477 - 9.526 $\mu$m, or more preferably a wavelength of 9.5168 $\mu$m, 9.5042 $\mu$m, or 9.4861 $\mu$m. By modulating the wavelength modulation range in this way, it is possible to reduce any interference effects from ethylene ($C_2H_4$), ammonia ($NH_3$), and/or carbon dioxide ($COz$), and to thereby improve the concentration measurement accuracy when measuring a low concentration of methanol ($CH_3OH$). Note that in a case in which methanol is being measured, it is necessary that the pressure inside the cell 1 be decreased to 15 kPa or less.

The temperature adjustment control unit 72 controls the current source (or voltage source) of the temperature adjustment unit 3 by outputting a control signal that causes the temperature adjustment unit 3 to maintain a predetermined target temperature. As a result, the temperature adjustment unit 3 adjusts the temperature of the semiconductor laser 2 such that the semiconductor laser 2 maintains a predetermined target temperature.

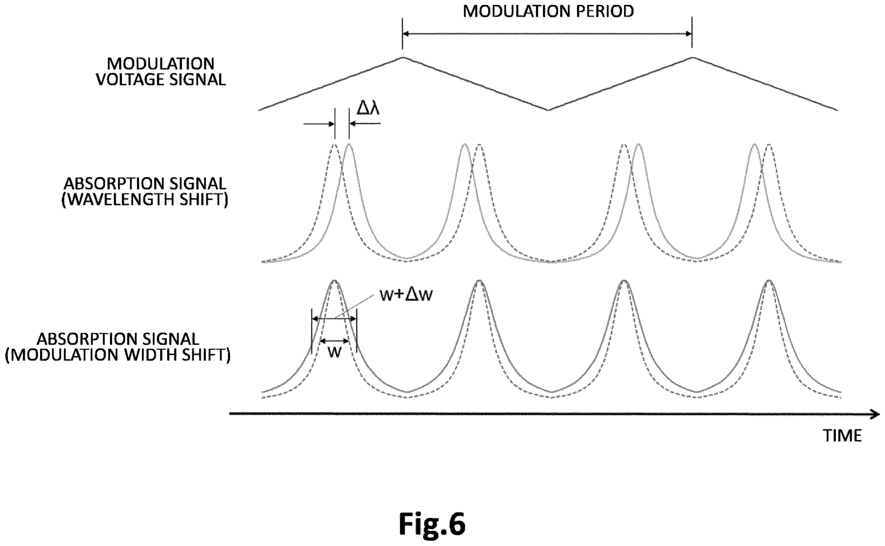

In addition, the control unit 7 of the present embodiment is provided with a relational data storage unit 73 that stores wavelength correction relational data showing a relationship between the ambient temperature of the semiconductor laser 2 and a correction parameter P ($\Delta\lambda$) (see FIG. 6) that is used to correct a wavelength shift relative to a target wavelength that is used to measure a measurement target component in the semiconductor laser 2, and modulation correction relational data showing a relationship between this ambient temperature and a correction parameter P (Δw) (see FIG. 6) that is used to correct a modulation width shift in the semiconductor laser 2.

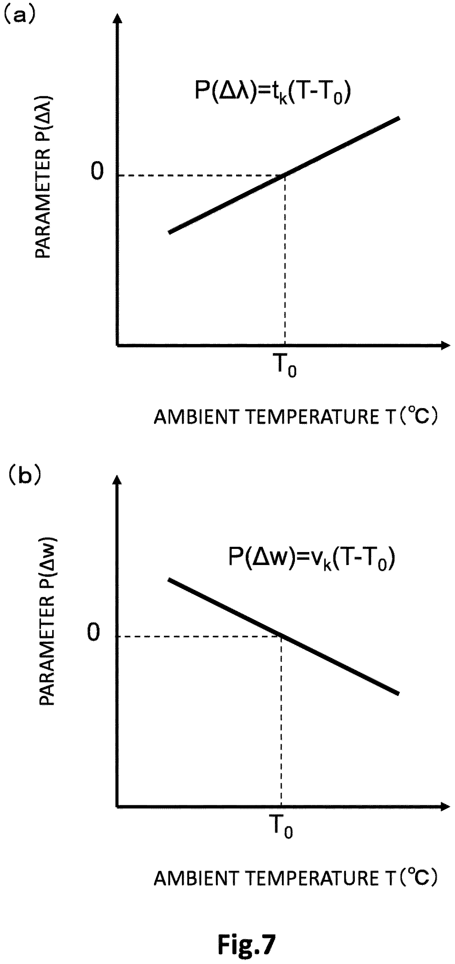

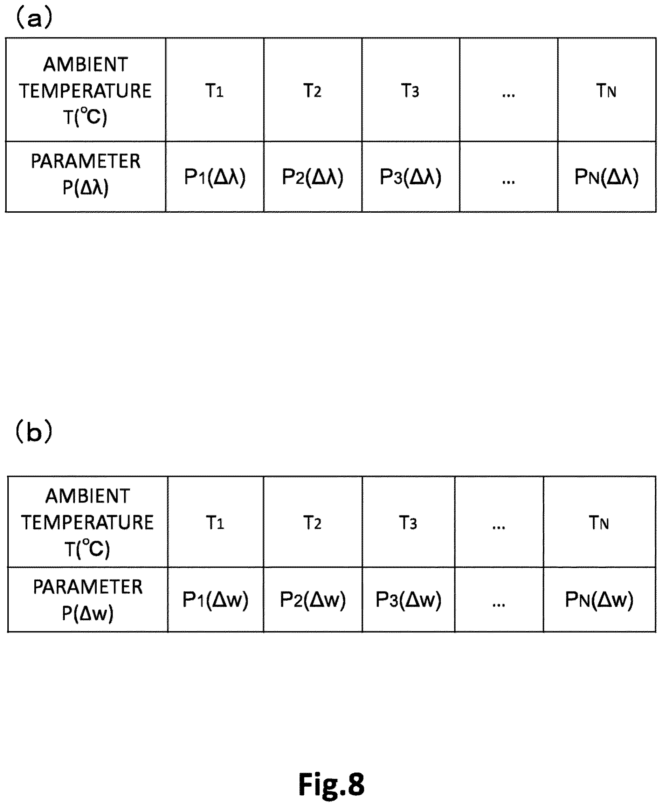

The wavelength correction relational data is shown in FIG. 7 (a). This wavelength correction relational data is created by determining, in advance, by means of experiment or by means of calculation an amount of change in a target temperature which is a required parameter P (Δλ) that is needed in order to correct wavelength shift in the semiconductor laser 2 at each ambient temperature of the semiconductor laser 2. In FIG. 7 (a), P (Δλ) is an amount of change in a target temperature, $T_0$ is a reference temperature (for example, room temperature (25 °C)), and $t_k$ is a coefficient showing a degree of influence of the amount of change in a target temperature at an ambient temperature T relative to the reference temperature $T_0$. As is shown in FIG. 7 (a), the wavelength correction relational data may be in the form of an equation, or as is shown in FIG. 8 (a), may be in the form of a lookup table.

The modulation correction relational data is shown in FIG. 7 (b). This modulation correction relational data is created by determining, in advance, by means of experiment or by means of calculation an amount of change in a drive voltage (or current) which is a required parameter P (Δw) that is needed in order to correct modulation width shift in the semiconductor laser 2 at each ambient temperature of the semiconductor laser 2. In FIG. 7 (b), P (Δw) is an amount of change in a drive voltage (or current), $T_0$ is a reference temperature (for example, room temperature (25 °C)), and $v_k$ is a coefficient showing a degree of influence of the amount of change in the drive voltage (or current) at the ambient temperature T relative to the reference temperature $T_0$. As is shown in FIG. 7 (b), the modulation correction relational data may be in the form of an equation, or as is shown in FIG. 8 (b), may be in the form of a lookup table.

The temperature adjustment control unit 72 corrects wavelength shift in the semiconductor laser 2 by altering the target temperature of the temperature adjustment unit 3 using detection temperatures obtained by the temperature sensor 4 and the wavelength correction relational data. Moreover, the light source control unit 71 corrects the modulation width of the semiconductor laser 2 by altering the drive voltage or the drive current of the semiconductor laser 2 using detection temperatures obtained by the temperature sensor 4 and the modulation correction relational data. More specifically, the light source control unit 71 corrects the modulation width by adjusting the offset or amplitude of the modulation voltage (or modulation current) in order to modulate the wavelength.

The signal processing unit 8 is formed by a logarithm calculation unit 81, a correlation value calculation unit 82, a storage unit 83, a wavelength shift determination unit 84, and a concentration calculation unit 85 and the like.

The logarithm calculation unit 81 performs a logarithmic calculation on a light intensity signal which is an output signal from the photodetector 5. A function I (t) showing changes over time in the light intensity signal obtained by the photodetector 5 takes the form shown by 'light intensity I (t)' in FIG. 5, and as a result of a logarithmic calculation being performed, this function I (t) then takes the form shown by 'logarithmic intensity L (t)' in FIG. 5.

The correlation value calculation unit 82 calculates respective correlational values between intensity related signals that relate to the intensity of sample light, and a plurality of predetermined characteristic signals. These characteristic signals are signals that are used to extract waveform characteristics of intensity related signals by obtaining a correlation thereof with the intensity related signals. Signals that may be used as the characteristic signals include, for example, sinusoidal wave signals, and various signals that match the waveform characteristics that a user wishes to extract from other intensity related signals.

Hereinafter, an example of a case in which a signal that is not a sinusoidal signal is used as the characteristic signal will be described. The correlation value calculation unit 82 calculates respective correlation values between an intensity related signal relating to the intensity of a sample light, and a plurality of characteristic signals from which different correlations are obtained than from a sinusoidal wave signal (i.e., a sine function) relative to this intensity related signal. Here, the correlation value calculation unit 82 uses a light intensity signal that has been calculated logarithmically (i.e., logarithmic intensity L (t)) as the intensity related signal.

The correction value calculation unit 82 employs the following Equation 1 in order to calculate a plurality of sample correlation values Si which are the respective correlation values between the intensity related signal of the sample light, and each of the plurality of characteristic signals using a number of characteristic signals Fi (t) (wherein i = 1, 2, ..., n) that is more than a number obtained by adding the number of types of measurement target component to the number of types of interference components whose interference effects need to be removed. Note that T in Equation 1 given below is the modulation period.

[Equation 1]

$$S_i = \int_{-T/2}^{T/2} L(t) \cdot F_i(t)\, dt \quad (i = 1,2,\cdots,n)$$

$$R_i = \int_{-T/2}^{T/2} L_0(t) \cdot F_i(t)\, dt \quad (i = 1,2,\cdots,n)$$

When calculating a sample correlation value, as in the above Equation 1, it is desirable that the correlation value calculation unit 82 calculate sample correlation values $S'_i$ that have been corrected by subtracting reference correlation values $R_i$, which are correlation values between the intensity related signal $L_0$ (t) of the reference light and the plurality of characteristic signals $F_i$ (t), from the correlation values Si between the intensity related signal L (t) of the sample light and the plurality of characteristic signals Fi (t). By employing this method, any offset contained in the sample correlation values is removed so that the correlation values become proportional to the concentrations of the measurement target component and the interference components, and it becomes possible to reduce measurement errors. Note that it is also possible to employ a structure in which reference correlation values are not subtracted.

Here, the acquisition timing when the reference light is acquired may be simultaneous with the acquisition of the sample light, either before or after the sample light is acquired, or may be another desired timing. It is also possible for the intensity related signal of the reference light or the reference correlation values to be acquired in advance and stored in the storage unit 83. Moreover, a method for simultaneously acquiring the reference light that may be considered is one in which, for example, two photodetectors 5 are provided. Modulation light from the semiconductor laser 2 is then split using a beam splitter or the like. One of these is used for the sample light measurement, while the other is used for the reference light measurement.

In the present embodiment, the correlation value calculation unit 82 uses, as the plurality of characteristic signals Fi (t), a function that enables the waveform characteristics of the logarithmic intensity L (t) to be ascertained more easily than a sine function. In a sample gas that includes the measurement target component and one interference component, in a case in which a user desires to perform further corrections of the effects of wavelength shift of the reference light, a method in which three characteristic signals Fi (t), $F_2$ (t), and $F_3$ (t) are used may be considered. As these three characteristic signals, using a function based on a Laurent function whose shape is similar to that of an absorption spectrum such as that shown below in Equation 2, or a partial differential function for a shift from a base time position of a function that is based on this Laurent function may be considered. Note that w in Equation 2 is a Laurent width, s is a shift from a base time position of an absorption peak that is caused by a wavelength shift, A is an arbitrary constant, and $A_1$, $A_2$, and $A_3$ are offsets that, when $F_1$ (t), $F_2$ (t), and $F_3$ (t) are each integrated at the modulation period, are adjusted so as to enable these to become zero. If functions such as these are used for the characteristic signals, then it is possible for spectrum changes caused by the effects of wavelength shift of the reference light to be ascertained with a greater degree of sensitivity, and it becomes possible to make more accurate corrections of the effects of wavelength shift of the reference light. Moreover, as the characteristic signals, instead of using a function that is based on a Laurent function, it is also possible to use a function that is based on a Voigt function, or a function that is based on a Gaussian function, or the like. By using this type of function for the characteristic signals, it is possible to obtain greater correlation values than when a sine function is used, and to thereby improve the measurement accuracy.

[Equation 2]

$$F_1(t) = \frac{A}{1 + \left(\dfrac{|t| - s_1}{w_1}\right)^2} - A_1 \quad \left(-\frac{T}{2} \le t \le \frac{T}{2}\right)$$

$$F_2(t) = \frac{A}{1 + \left(\dfrac{|t| - s_2}{w_2}\right)^2} - A_2 \quad \left(-\frac{T}{2} \le t \le \frac{T}{2}\right)$$

$$F_3(t) = \frac{\partial F_1}{\partial s_1} - A_3 \quad \left(-\frac{T}{2} \le t \le \frac{T}{2}\right)$$

The storage unit 83 stores independent correlation values which are correlation values per unit concentration for the measurement target component and the respective interference components determined from the respective intensity related signals in cases in which a measurement target component and various interference components are independently present in a known reference light wavelength shift amount, and from the plurality of characteristic signals Fi (t). The plurality of characteristic signals Fi (t) that are used to determine these independent correlation values are the same as the plurality of characteristic signals Fi (t) used by the correlation value calculation unit 82. In this way, independent correlation values for each wavelength shift of various reference lights are stored in the storage unit 83.

Here, when the storage unit 83 is storing the independent correlation values, it is desirable that the reference correlation values are firstly subtracted from the correlation values when the measurement target component and the respective interference components are present independently, and that the storage unit 83 then store independent correlation values that have undergone correction by being converted into per unit concentrations. By employing this method, any offset contained in the independent correlation values is removed so that the correlation values become proportional to the concentrations of the measurement target component and the interference components, and it becomes possible to reduce measurement errors. Note that it is also possible to employ a structure in which reference correlation values are not subtracted.

The wavelength shift determination unit 84 determines a wavelength shift amount W of the reference light from a light intensity signal that is output from the photodetector 5.

For example, the following procedure may be considered as the determination method used to determine the wavelength shift amount W.

(a) The respective independent correlation values $s_{itar}$ ($W_k$) and $s_{iint}$ ($W_k$) that correspond to the respective characteristic signals $F_i(t)$ of the interference components and the measurement target component in the wavelength shifts $W_k$ (wherein k = 1, 2, ..., I) of each reference light are acquired in advance, and the wavelength shift W of the reference light is then determined by comparing and collating the sample correlation values obtained at the time of measurement and the aforementioned independent correlation values. As the specific method employed for this comparison and collation, a non-linear least squares method in conjunction with an iterative calculation employing, for example, a steepest descent method, a Gauss-Newton method, or a Levenberg-Marquardt method or the like may be considered. In the case of this method, the number of characteristic signals that are required is equal to or greater than a number obtained by adding 1 to the sum of the number of types of measurement target components and the number of types of interference components. The reason why 1 is added is so as to enable the number to be used for a wavelength shift amount that is a parameter common to the light absorption spectrums of each component.

(b) The wavelength shift amount W of the reference light is determined using relational data showing a relationship between the ambient temperature and the wavelength shift amount W, and the measured ambient temperature. At this time, the relational data is created by determining in advance via experiment or via calculation the wavelength shift amounts W of the reference light at each ambient temperature of the light source 2.

The concentration calculation unit 85 calculates the concentration of a measurement target component using the plurality of sample correlation values obtained by the correlation value calculation unit 82.

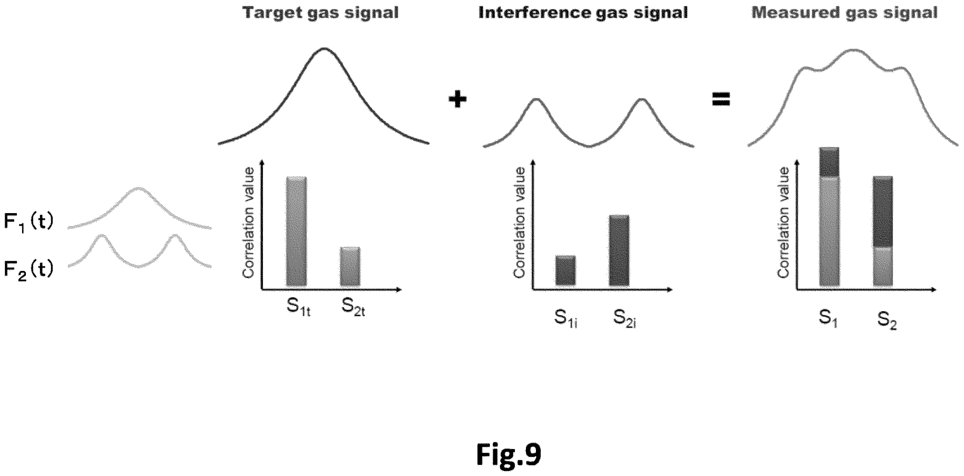

More specifically, the concentration calculation unit 85 calculates the concentration of a measurement target component based on a plurality of sample correlation values obtained by the correlation value calculation unit 82, a wavelength shift amount W determined by the wavelength shift determination unit 84, and a plurality of independent correlation values stored in the storage unit 83. More specifically, the concentration calculation unit 85 corrects a plurality of independent correlation values stored in the storage unit 83 from the wavelength shift amount W obtained by the wavelength shift determination unit 84, and acquires the results. Next, the concentration calculation unit 85 calculates the concentration of the measurement target component by solving simultaneous equations formed by the plurality of sample correlation values obtained by the correlation value calculation unit 82, the corrected plurality of independent correlation values corresponding to the determined wavelength shift amount W, and the respective concentrations for each interference component and the measurement target component (see FIG. 9).

Next, an example of an operation of the analysis device 100 will be described in combination with a detailed description of each of the above-described units. In the description below a case is hypothesized in which a single measurement target component and a single interference component are contained in the sample gas.

[Reference Measurement]

In a state in which the ambient temperature is kept constant at a base temperature $T_0$ (for example, room temperature: 25 °C), firstly, the light source control unit 71 controls the semiconductor laser 2 so that the wavelength of the laser light is modulated at a predetermined modulation frequency and modulation depth, and so as to be centered on a peak of the absorption spectrum of the measurement target component. Note that it is also possible to perform the measurement of the reference correlation values by performing reference measurements using a zero gas prior to performing the reference measurements using a span gas.

Next, the span gas (i.e., a gas having a known component concentration) is introduced into the cell 1 either by an operator or automatically, and the reference measurement is performed. This reference measurement is performed respectively in a span gas in which the measurement target component is present independently, and in a span gas in which the interference component is present independently.

More specifically, in the reference measurement, the logarithm calculation unit 61 receives the respective output signals from the photodetector 5 in each wavelength shift amount of the reference light, and then calculates the logarithmic intensity L (t). Next, the correlation value calculation unit 82 calculates correlation values between this logarithmic intensity L (t) and the three characteristic signals Fi (t), $F_2$ (t), and $F_3$ (t), and divides a result obtained when a reference correlation value is subtracted from these correlation values by the concentration of the span gas. As a result, independent correlation values which are the correlation values for each span gas per unit concentration are calculated. Note that, instead of calculating independent correlation values, it is also possible to store relationships between span gas concentrations and the correlation value relevant to each span gas.

This will now be described in more detail.

The wavelength shift amount of the reference light is adjusted to $w_k$, and a span gas in which the measurement target component is present independently is introduced into the interior of the cell 1. Correlation values $S_{1tar}$ ($w_k$), $S_{2tar}$ ($w_k$), and $S_{3tar}$ ($w_k$) of the measurement target component are then calculated by the correlation value calculation unit 82. Here, $S_{1tar}$ ($w_k$) is a correlation value with the first characteristic signal, $S_{2tar}$ ($w_k$) is a correlation value with the second characteristic signal, and $S_{3tar}$ ($w_k$) is a correlation value with the third characteristic signal. Next, the correlation value calculation unit 82 divides a result obtained when a reference correlation value Ri is subtracted from these correlation values $S_{1tar}$ ($w_k$), $S_{2tar}$ ($w_k$), and $S_{3tar}$ ($w_k$) by the span gas concentration $c_{tar}$ of the measurement target component. As a result, the independent correlation values $s_{1tar}$ ($w_k$), $s_{2tar}$ ($w_k$), and $s_{3tar}$ ($w_k$) are calculated. This procedure is performed in each wavelength shift amount using a method such as changing the set temperature of the semiconductor laser 2, while sequentially changing the wavelength shift amount of the reference light (for example, by -0.01 cm$^{-1}$ ~ +0.01 cm$^{-1}$ for each 0.001 cm$^{-1}$), and the relationships between the independent correlation values and the wavelength shift amount thereof in each obtained wavelength shift amount are stored. Note that the span gas concentration $c_{tar}$ of the measurement target component is input in advance into the signal processing unit 8 by a user or the like.

Moreover, the wavelength shift amount of the reference light is adjusted to $w_k$, and a span gas in which the interference component is present independently is introduced into the interior of the cell 1. Correlation values $S_{1int}$ ($w_k$), $S_{2int}$ ($w_k$), and $S_{3int}$ ($w_k$) of the interference component are then calculated by the correlation value calculation unit 82. Here, $S_{1int}$ ($w_k$) is a correlation value with the first characteristic signal, $S_{2int}$ ($w_k$) is a correlation value with the second characteristic signal, and $S_{3int}$ ($w_k$) is a correlation value with the third characteristic signal. Next, the correlation value calculation unit 82 divides a result obtained when the reference correlation value Ri is subtracted from these correlation values $S_{1int}$ ($w_k$), $S_{2int}$ ($w_k$), and $S_{3int}$ ($w_k$) by the span gas concentration $c_{int}$ of the interference component. As a result, the correlation value calculation unit 82 calculates the independent correlation values $s_{1int}$ ($w_k$), $s_{2int}$ ($w_k$), and $s_{3int}$ ($w_k$).

This procedure is performed in each wavelength shift amount using a method such as changing the set temperature of the semiconductor laser 2, while sequentially changing the wavelength shift amount of the reference light (for example, by -0.01 cm$^{-1}$ ~ +0.01 cm$^{-1}$ for each 0.001 cm$^{-1}$), and the relationships between the independent correlation values and the wavelength shift amount thereof in each obtained wavelength shift amount are stored. Note that the span gas concentration $c_{int}$ of the interference component is input in advance into the signal processing unit 8 by a user or the like.

The independent correlation values sitar ($w_k$), $S_{2tar}$ ($w_k$), $S_{3tar}$ ($w_k$), $s_{1int}$ ($w_k$), $s_{2int}$ ($w_k$), and $s_{3int}$ ($w_k$) in the wavelength shift amounts $w_k$ of each reference light calculated using the above-described method are stored in the storage unit 83. Note that this reference measurement may be performed prior to product shipment, or it may be performed at regular intervals.

[Sample Measurement]

The light source control unit 71 controls the semiconductor laser 2 so that the wavelength of the laser light is modulated at a predetermined modulation frequency and modulation depth, and so as to be centered on a peak of the absorption spectrum of the measurement target component. Here, using detection temperatures obtained by the temperature sensor 4 and wavelength correction relational data, the temperature control unit 72 alters the target temperature of the temperature adjustment unit 3 so as to correct any wavelength shift of the semiconductor laser 2. In addition, using detection temperatures obtained by the temperature sensor 4 and modulation correction relational data, the light source control unit 71 alters the drive voltage or drive current of the semiconductor laser 2 so as to correct the modulation width of the semiconductor laser 2.

Next, a sample gas is introduced into the interior of the cell 1 either by an operator or automatically, and a sample measurement is performed.

More specifically, in this sample measurement, the logarithm calculation unit 81 receives the output signals from the photodetector 3, and then calculates the logarithmic intensity L (t). Next, the correlation value calculation unit 82 calculates sample correlation values Si, $S_2$, and $S_3$ between this logarithmic intensity L (t) and the plurality of characteristic signals Fi (t), $F_2$ (t), and $F_3$ (t), and then calculates sample correlation values S'i and S'$_2$ that are obtained when the reference correlation value Ri is subtracted from these correlation values.

In addition, the wavelength shift determination unit 84 determines the wavelength shift amount W using the above-described method.

Using the independent correlation values in the wavelength shift amount $w_k$ of each reference light stored in the storage unit 83, and the wavelength shift amount W determined by the wavelength shift determination unit 84, the concentration calculation unit 85 determines independent correlation values s'$_{1tar}$, s'$_{2tar}$, s'$_{1int}$, and s'$_{2int}$ of the measurement target components and the interference components that have been corrected using the wavelength shift amount W. A method employing, for example, linear interpolation, secondary interpolation, or spline interpolation or the like might be considered for this determination.

Next, the concentration calculation unit 85 solves the following two-dimensional simultaneous equations formed by the sample correlation values S'$_1$ and S'$_2$ corrected using the reference correlation values calculated by the correlation value calculation unit 82, the corrected independent correlation values s'$_{1tar}$, s'$_{2tar}$, s'$_{1int}$, and s'$_{2int}$, and the concentrations $C_{tar}$ and $C_{int}$ for each of the measurement target component and the respective interference components (see FIG. 9).

[Equation 3]

$$s'_{1tar} \cdot C_{tar} + s'_{1int} \cdot C_{int} = S'_1$$
$$s'_{2tar} \cdot C_{tar} + s'_{2int} \cdot C_{int} = S'_2$$

Note that, even in a case in which two or more interference components are assumed to be present, by adding the same number of independent correlation values as the number of interference components, and solving the same original number of simultaneous equations as the number of types of components, it is still possible, in the same way, to determine the concentration of a measurement target component from which interference effects and coexistence effects have been removed.

In other words, generally, in a case in which the measurement target component and the interference components are combined to give n number of gas types that are present, if a corrected independent correlation value of a j-th gas type in an i-th characteristic signal is taken as s'$_{ij}$, a concentration of the j-th gas type is taken as $C_j$, and a sample correlation value in an i-th characteristic signal Fi (t) is taken as Si, then the following Equation 4 is established.

[Equation 4]

$$s'_{11}C_1 + s'_{12}C_2 + s'_{13}C_3 + \cdots + s'_{1n}C_n = S'_1$$
$$s'_{21}C_1 + s'_{22}C_2 + s'_{23}C_3 + \cdots + s'_{2n}C_n = S'_2$$
$$s'_{31}C_1 + s'_{32}C_2 + s'_{33}C_3 + \cdots + s'_{3n}C_n = S'_3$$
$$\vdots$$
$$s'_{n1}C_1 + s'_{n2}C_2 + s'_{n3}C_3 + \cdots + s'_{nn}C_n = S'_n$$

By solving simultaneous equations with n variables represented in this Equation 4, it is possible to determine concentrations in which the interference effects of each gas of the measurement target component and the interference components have been corrected. Note that even in a case in which interference components are not included in a sample, by solving the simultaneous equations with n variables given above, it is still possible to determine concentrations in which the interference effects of each gas of the measurement target component and the interference components have been corrected.

[Effects of the Present Embodiment]

According to the analysis device 100 of the present embodiment that is formed in the manner described above, it is possible to accurately measure a concentration of a measurement target component in the form of at least one of carbon dioxide ($CO_2$), carbon monoxide (CO), ethylene ($C_2H_4$), ethane ($C_2H_6$), water ($H_2O$), acetylene ($C_2H_2$), and methane ($CH_4$) in a processing gas.

Moreover, using first relational data showing a relationship between the ambient temperature of the laser light source 2 and a parameter that is used to correct a wavelength shift in the laser light source 2, a target temperature of the temperature adjustment unit 3 is altered from a detection temperature from the temperature sensor 4 that detects the ambient temperature around the laser light source 2. Because of this, it is possible to reduce changes in an oscillation wavelength of a laser light source that are caused by fluctuations in the ambient temperature. As a result, it is possible to reduce changes in an absorption spectrum that are caused by fluctuations in a laser light source without having to use a reference cell into which a reference gas has been injected, and to thereby enable a concentration of a measurement target component to be measured accurately.

In particular, in the present embodiment, because any wavelength shift caused by fluctuations in the ambient temperature as well as the modulation width are corrected, a wavelength modulation range in a case in which the concentrations of each of carbon dioxide ($CO_2$), carbon monoxide (CO), ethylene ($C_2H_4$), ethane ($C_2H_6$), water ($H_2O$), acetylene ($C_2H_2$), or methane ($CH_4$) that are present in a processing gas are measured can be accurately set, and the concentrations of each of these can be accurately measured.

Moreover, in addition to the above-described physical wavelength shift correction, because the amount W of wavelength shift in the reference light is determined via calculation, and, using this determined wavelength shift amount W, the concentration of a measurement target component in which the effects of reference light wavelength shift have been further corrected is calculated, it is possible to correct changes in a light absorption spectrum of a measurement target component that are generated by the reference light wavelength shift and that cannot be suppressed solely by a physical wavelength shift correction, and to thereby measure the concentration of a measurement target component even more accurately.

In addition, according to the analysis device 100 of the present embodiment, because the logarithmic intensity L (t), which is an intensity related signal relating to the intensity of sample light, and respective correlational values Si of the plurality of characteristic signals Fi (t) relative to this logarithmic intensity L (t) are calculated, and the concentration of a measurement target component is calculated using the calculated plurality of correlational values Si, it is possible to ascertain characteristics of an absorption signal using dramatically fewer variables without having to convert an absorption signal into an absorption spectrum, and to measure the concentration of a measurement target component via a simple calculation without having to perform complex spectrum calculation processing. For example, it is necessary for several hundred or more data points to be used in normal spectrum fitting, however, if the present invention is employed it is possible to calculate a concentration to an equivalent level of accuracy using at most between approximately several and several tens of correlation values. As a result, it is possible to dramatically reduce the calculation processing load so that a sophisticated calculation processing device is rendered unnecessary, and both the cost and the size of the analysis device 100 can be reduced.

Here, because signals that enable different correlations than those from a sinusoidal signal to be obtained are used for the plurality of characteristic signals, it is possible to determine the concentration of a measurement target component at an equivalent or greater accuracy as that obtained by using an analysis device that performs concentration calculations using a conventional lock-in detection method.

[Additional Embodiments]

For example, the logarithm calculation unit 61 in each of the above-described embodiments performs a logarithm calculation on a light intensity signal from the photodetector 3, however, it is also possible to calculate a logarithm of a ratio between the sample light intensity and the modulation light (i.e., reference light) intensity (also known as the light absorbance) using a light intensity signal from the photodetector 3. At this time, it is also possible for the logarithm calculation unit 61 to calculate a logarithm of the sample light intensity and, after calculating a logarithm of the intensity of the reference light, to subtract these so as to calculate the light absorbance. In addition, it is also possible to calculate the light absorbance by firstly determining a ratio between the sample light intensity and the reference light intensity, and then acquiring a logarithm of this ratio.

Moreover, the correlation value calculation unit 62 of each of the above-described embodiments calculates a correlation value between an intensity related signal and a characteristic signal, however, it is also possible to calculate an inner product value of an intensity related signal and a characteristic signal.

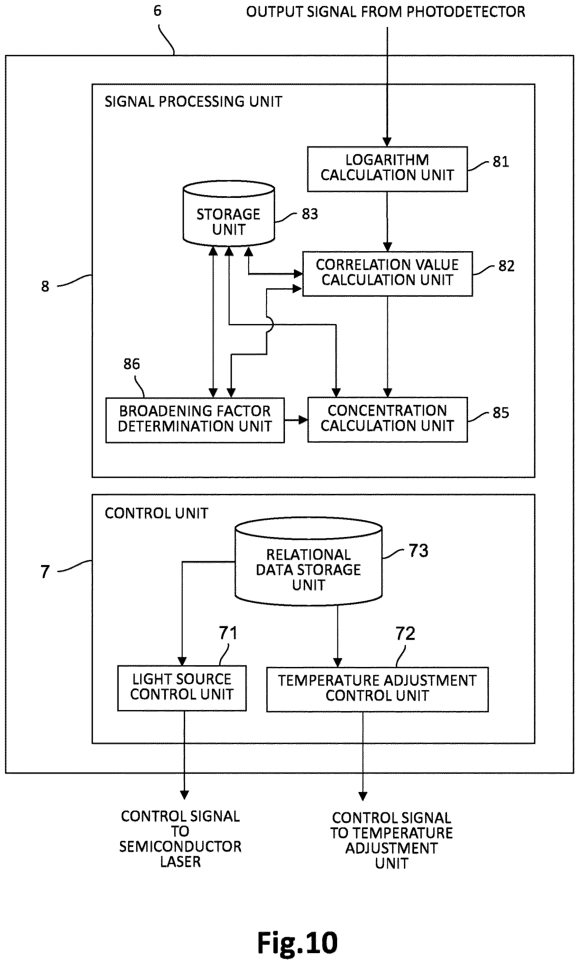

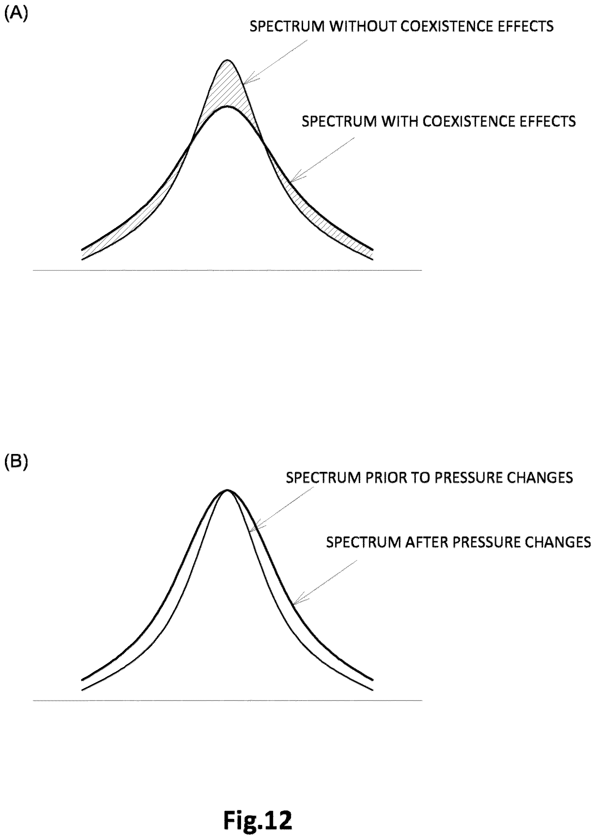

Furthermore, in addition to the function of the analysis device 100 of the above-described embodiments of correcting wavelength shift, or else instead of this function of correcting wavelength shift, it is also possible for the analysis device 100 to have a function of correcting broadening that is caused by coexistence effects (see FIG. 12). In this case, as is shown in FIG. 10, the signal processing unit 8 of the analysis device 100 is provided with a broadening factor determination unit 86 that determines a broadening factor showing a rate of change in a light absorption spectrum of the measurement target component or an interference component that is generated by coexistence components contained in a sample.

The broadening factor determination unit 86 determines a broadening factor $F_B$ showing a rate of change in a light absorption spectrum of the measurement target component or an interference component that is generated by a coexistence component contained in a sample. Note that, in a case in which a coexistence effect caused by a coexistence component on an interference component also needs to be considered, then the broadening factor $F_B$ is added and determined for each individual component.

A method used to determine the broadening factor $F_B$ that may be considered is, for example, the procedure described in either (a) or (b) below.

(a) The respective independent correlation values $s_{itar}(p_k)$ and $s_{iint}(p_k)$ corresponding to the respective characteristic signals Fi (t) of the measurement target component and interference components at each pressure $p_k$ (wherein k = 1, 2, ..., l) inside a cell are acquired in advance. The sample correlation values obtained at the time of measurement and the independent correlation values are then compared and collated, and the broadening factor $F_B$ is thereby determined. Note that, when performing the comparison and collation, the independent correlation values are converted using pressure values from inside the cell and the relationship given below in Equation 5. In a case in which this method is used, the number of characteristic signals required is a number that is equal to or greater than the sum of the number of types of measurement target component, the number of types of interference components, and the number of types of broadening factors.

[Equation 5]

$$s'_{ij} = \frac{s_{ij}(F_B \cdot p)}{F_B}$$

Here, p is the pressure of the sample as measured by the pressure sensor 7, $F_B$ is the broadening factor determined by the broadening factor determination unit 86, $s_{ij}$ is an independent correlation value for each pressure that is stored in the storage unit 63, and $s'_{ij}$ is a corrected independent correlation value. Note that Equation 5 above shows a formula for determining the corrected independent correlation value $s'_{ij}$ by multiplying by $1/F_B$ the independent correlation value at a pressure obtained when the pressure is multiplied by $F_B$ for the independent correlation values $s_{ij}(p)$ at the pressure p of the sample at the time the sample was measured.

Note also that, in a case in which an interference component is also affected by broadening due to a coexistence component, then it is also possible to determine the broadening factor of the interference component separately, and to then correct the independent correlation value of the interference component. By doing this, it is possible to further improve the measurement accuracy.

(b) The broadening factor $F_B$ is determined using relational data showing a relationship between the concentration of a coexistence component and the broadening factor $F_B$, and using the measured concentration of the coexistence component.

At this time, the relational data is created by determining in advance, either by experiment or by calculation, the broadening factor $F_B$ at each concentration of the coexistence component. The measured concentration of the coexistence component may be measured by the analysis device 100 of the present embodiment prior to the correction of the coexistence component, or may be measured using a separate analysis device.

The concentration calculation unit 65 calculates the concentration of a measurement target component using a plurality of sample correlation values obtained by the correlation value calculation unit 62.

More specifically, the concentration calculation unit 65 calculates the concentration of the measurement target component based on a plurality of sample correlation values obtained by the correlation value calculation unit 62, the broadening factor $F_B$ determined by the broadening factor determination unit 64, and the plurality of independent correlation values stored in the storage unit 63. Still more specifically, the concentration calculation unit 65 corrects and then acquires the plurality of independent correlation values stored in the storage unit 63 from the broadening factor $F_B$ obtained by the broadening factor determination unit 64. Next, the concentration calculation unit 65 calculates the concentration of the measurement target component by solving simultaneous equations formed by the plurality of sample correlation values obtained by the correlation value calculation unit 62, the corrected plurality of independent correlation values corresponding to the determined broadening factor $F_B$, and the concentrations of the component being determined and of each of the respective interference components.

Even more specifically, using independent correlation values at the pressure $p_k$ within each cell stored in the storage unit 63, the pressure values p within the cells measured by the pressure sensor 7, the broadening factor $F_B$ determined by the broadening factor determination unit 64, and the above-described Equation 5, the concentration calculation unit 65 determines independent correlation values $s'_{1tar}$ and $s'_{2tar}$ of the measurement target component that have been corrected using both the pressure within the cell and the broadening factor, and also independent correlation values $s'_{1int}$ and $s'_{2int}$ of the interference components that have been corrected using only the pressure within the cell (i.e., the broadening factor is taken as 1). A method employing, for example, linear interpolation, secondary interpolation, or spline interpolation or the like might be considered for this determination.

Next, the concentration calculation unit 65 solves the simultaneous equations given below that are formed by the sample correlation values $S'_1$ and $S'_2$ corrected using the reference correlation values calculated by the correlation value calculation unit 62, the corrected independent correlation values s'itar, s'itar, $s'_{1int}$, and $s'_{2int}$, and the concentrations $C_{tar}$ and $C_{int}$ for the measurement target component and the respective interference components.

[Equation 6]

$$s'_{1tar} \cdot C_{tar} + s'_{1int} \cdot C_{int} = S'_1$$

$$s'_{2tar} \cdot C_{tar} + s'_{2int} \cdot C_{int} = S'_2$$

If this method is employed, then by performing a simple and reliable arithmetic operation, namely, by solving the simultaneous equations given in the above Equation 6, it is possible to determine the concentration $C_{tar}$ of a measurement target component from which interference effects and coexistence effects have been removed. If this structure is employed, then because it is possible, using the modulation width correction of the laser light source 2 of the present invention, to suppress changes in the modulation width of a laser light source that are caused by fluctuations in the ambient temperature, and to accurately correct broadening that is caused by coexistence effects, it becomes possible to measure the concentration of a measurement target component even more accurately.

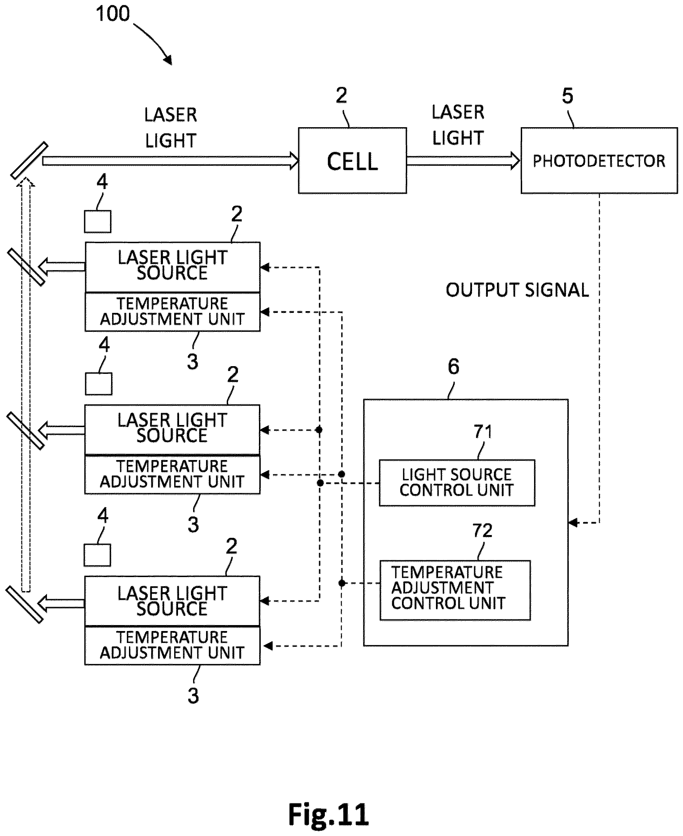

Moreover, as is shown in FIG. 11, it is also possible for the analysis device 100 to be provided with a plurality of laser light sources 2 that irradiate laser light onto the cell 1, and with a plurality of temperature adjustment units 3 that correspond to these laser light sources 2. A plurality of laser light sources 2 that correspond, for example, to the measurement target components illustrated in the above-described embodiments may be considered. Here, in the analysis device 100, the plurality of laser light sources 2 are pulse-oscillated by the light source control unit 71 at the same oscillation period as each other, but at different oscillation timings than each other. Here, the control contents of the light source control unit 71 and the temperature adjustment control unit 72 with regard to each laser light source 2 and each temperature adjustment unit 3 are the same as in the above-described embodiments. The signal processing device 6 separates the respective signals of the plurality of laser light sources 2 from the light intensity signal obtained by the photodetector 5, and using the separated light intensity signals from each of the laser light sources 2, calculates the concentration of the measurement target components that correspond to each of the respective laser light sources 2. Note that the calculation of the concentration of a measurement target component by the signal processing unit 8 is the same as in the above-described embodiments.

In the above-described embodiments, a structure in which not only is wavelength shift corrected physically, but wavelength shift is also corrected by means of calculation, however, it is also possible to employ a structure in which correcting wavelength shift by means of calculation is not performed. Alternatively, it is also possible to correct wavelength shift by means of calculation without correcting the wavelength shift physically, or to neither correct wavelength shift physically nor to correct wavelength shift by means of calculation.

Moreover, in the above-described embodiments, not only is wavelength shift that is caused by the ambient temperature corrected, but shifting of the modulation width is also corrected, however, it is also possible to employ a structure in which shifting of the modulation width is not corrected.

In addition, in each of the above-described embodiments, the storage unit 83 stores independent correlation values that have been corrected using reference correlation values, however, it is also possible to employ a structure in which independent correlation values are stored in a storage unit 883 prior to their being corrected, and in which after firstly subtracting a reference correlation value from a pre-correction independent correlation value, the concentration calculation unit 83 then determines an independent correlation value that has been corrected by being converted into a per unit concentration.

The plurality of characteristic signals are not limited to those in the above-described embodiments, and it is sufficient if they are functions that are mutually different from each other. It is also possible for either a function showing a light intensity obtained when a span gas having a known concentration is supplied, or a waveform of either a logarithmic intensity or a light absorbance (i.e., a sample spectrum) to be used as a characteristic signal. Moreover, in a case in which the concentration of a single measurement target component is to be measured, it is sufficient if there is at least one characteristic signal.

Furthermore, in a case in which the measurement target component and the interference components are combined so as to give n number of gas types, then using a number of types of characteristic signals that is greater than n, it is also possible to determine a number of independent correlation values and sample correlation values that is greater than the number of gas types, and to create an original number of simultaneous equations that is greater than the number of gas types, and to then determine the concentration of each component using the least squares method. If this type of method is employed, then it is possible to determine a concentration having an even smaller degree of error with regard to the measurement noise as well.

The signal processing unit of the above-described embodiment performs the functions of a correlation value calculation unit that calculates correlation values present in a concentration of the measurement target component using an intensity related signal relating to the intensity of the sample light, and characteristic signals from which predetermined correlations relative to the intensity related signal have been obtained, and the functions of a concentration calculation unit that calculates a concentration of the measurement target component using correlation values obtained by the correlation value calculation unit, however, it is also possible for a different calculation method to be employed.

The light source is also not limited to being a semiconductor laser, and another type of laser may instead be used. In fact, any light source may be used provided that it is a single wavelength light source having a sufficient half-width to guarantee a sufficient measurement accuracy, and is capable of undergoing wavelength modulation.

[Industrial Applicability]

According to the present invention, it is possible to accurately measure a concentration of a measurement target component in the form of at least one of carbon dioxide, carbon monoxide, ethylene, ethane, water, acetylene, methane, ammonia, and methanol in a processing gas.

In addition to these, various other additions, omissions, substitutions, and modifications may be made insofar as they do not depart from the spirit or scope of the present invention.

[Description of the Reference Numerals]

100   Analysis Device
1     Cell
2     Laser Light Source (Semiconductor Laser)
3     Temperature Adjustment Unit
4     Temperature Sensor
5     Photodetector

6       Signal Processing Device
7       Control Unit
81      Logarithm Calculation Unit
82      Correlation Value Calculation Unit
83      Storage Unit
84      Wavelength Shift Determination Unit
85      Concentration Calculation Unit


**Claims**

1.  An analysis device that measures a concentration of a measurement target component in the form of at least one of carbon dioxide, carbon monoxide, ethylene, ethane, water, acetylene, methane, ammonia, or methanol that are present in a processing gas, comprising:

    a laser light source that irradiates reference light onto the processing gas;
    a photodetector that detects an intensity of sample light that is generated as a result of the reference light being transmitted through the processing gas; and
    a concentration calculation unit that calculates a concentration of the measurement target component based on an output signal from the photodetector, wherein,
    in a case in which a concentration of the carbon dioxide is being measured, the concentration calculation unit calculates this concentration based on a carbon dioxide absorption between 4.23 ~ 4.24 $\mu$m, or 4.34 ~ 4.35 $\mu$m,
    in a case in which a concentration of the carbon monoxide is being measured, the concentration calculation unit calculates this concentration based on a carbon monoxide absorption between 4.59 ~ 4.61 $\mu$m,
    in a case in which a concentration of the water is being measured, the concentration calculation unit calculates this concentration based on a water absorption between 5.89 ~ 6.12 $\mu$m,
    in a case in which a concentration of the acetylene is being measured, the concentration calculation unit calculates this concentration based on an acetylene absorption between 7.56 ~ 7.66 $\mu$m, or 7.27 ~ 7.81 $\mu$m,
    in a case in which a concentration of the methane is being measured, the concentration calculation unit calculates this concentration based on a methane absorption between 7.67 ~ 7.80 $\mu$m, or 8.10 ~ 8.14 $\mu$m,
    in a case in which a concentration of the ethylene is being measured, the concentration calculation unit calculates this concentration based on an ethylene absorption between 8.46 ~ 8.60 $\mu$m,
    in a case in which a concentration of the ethane is being measured, the concentration calculation unit calculates this concentration based on an ethane absorption between 6.13 ~ 6.14 $\mu$m, or 6.09 ~ 6.45 $\mu$m,
    in a case in which a concentration of the ammonia is being measured, the concentration calculation unit calculates this concentration based on an ammonia absorption between 6.06 ~ 6.25 $\mu$m, or 8.62 ~ 9.09 $\mu$m, and
    in a case in which a concentration of the methanol is being measured, the concentration calculation unit calculates this concentration based on a methanol absorption between 9.35 ~ 9.62 $\mu$m.

2.  The analysis device according to Claim 1, wherein,

    in a case in which a concentration of the carbon dioxide is being measured, the concentration calculation unit calculates this concentration based on a carbon dioxide absorption between 4.234 ~ 4.238 $\mu$m, or 4.342 ~ 4.347 $\mu$m,
    in a case in which a concentration of the carbon monoxide is being measured, the concentration calculation unit calculates this concentration based on a carbon monoxide absorption between 4.594 ~ 4.604 $\mu$m,
    in a case in which a concentration of the water is being measured, the concentration calculation unit calculates this concentration based on a water absorption between 5.896 ~ 5.934 $\mu$m, or 6.046 ~ 6.114 $\mu$m,
    in a case in which a concentration of the acetylene is being measured, the concentration calculation unit calculates this concentration based on an acetylene absorption between 7.378 - 7.638 $\mu$m, 7.378 ~ 7.603 $\mu$m, 7.629 ~ 7.683 $\mu$m, 7.594 ~ 7.651 $\mu$m, or 7.566 ~ 7.634 $\mu$m,
    in a case in which a concentration of the methane is being measured, the concentration calculation unit calculates this concentration based on a methane absorption between 7.670 - 7.792 $\mu$m, 8.107 ~ 8.139 $\mu$m, or 8.102 ~ 8.121 $\mu$m,
    in a case in which a concentration of the ethylene is being measured, the concentration calculation unit calculates this concentration based on an ethylene absorption between 8.464 ~ 8.599 $\mu$m,
    in a case in which a concentration of the ethane is being measured, the concentration calculation unit calculates this concentration based on an ethane absorption between 6.135 ~ 6.139 $\mu$m, or 6.463 ~ 6.619 $\mu$m,

in a case in which a concentration of the ammonia is being measured, the concentration calculation unit calculates this concentration based on an ammonia absorption between 6.141 ~ 6.153 $\mu$m, or 8.939 ~ 8.968 $\mu$m, and

in a case in which a concentration of the methanol is being measured, the concentration calculation unit calculates this concentration based on a methanol absorption between 9.477 ~ 9.526 $\mu$m.

3. The analysis device according to Claim 1 or 2, wherein,

in a case in which a concentration of the carbon dioxide is being measured, the concentration calculation unit calculates this concentration based on a carbon dioxide absorption of 4.2347 $\mu$m, 4.2371 $\mu$m, 4.3428 $\mu$m, or 4.3469 $\mu$m,

in a case in which a concentration of the carbon monoxide is being measured, the concentration calculation unit calculates this concentration based on a carbon monoxide absorption of 4.5950 $\mu$m, or 4.6024 $\mu$m,

in a case in which a concentration of the water is being measured, the concentration calculation unit calculates this concentration based on a water absorption of 5.8965 $\mu$m, 5.9353 $\mu$m, 6.0486 $\mu$m, or 6.1138 $\mu$m,

in a case in which a concentration of the acetylene is being measured, the concentration calculation unit calculates this concentration based on an acetylene absorption of 7.5966 $\mu$m, 7.6233 $\mu$m, 7.6501 $\mu$m, 7.5698 $\mu$m, 7.6367 $\mu$m, or 7.6231 $\mu$m,

in a case in which a concentration of the methane is being measured, the concentration calculation unit calculates this concentration based on a methane absorption of 7.6704 $\mu$m, 7.7914 $\mu$m, 8.1073 $\mu$m, 8.1381 $\mu$m, 8.1022 $\mu$m, or 8.1206 $\mu$m,

in a case in which a concentration of the ethylene is being measured, the concentration calculation unit calculates this concentration based on an ethylene absorption of 8.4647 $\mu$m, or 8.5981 $\mu$m,

in a case in which a concentration of the ethane is being measured, the concentration calculation unit calculates this concentration based on an ethane absorption of 6.1384 $\mu$m, 6.4673 $\mu$m, 6.5008 $\mu$m, 6.5624 $\mu$m, or 6.6145 $\mu$m,

in a case in which a concentration of the ammonia is being measured, the concentration calculation unit calculates this concentration based on an ammonia absorption of 6.1450 $\mu$m, 6.1487 $\mu$m, 6.1496 $\mu$m, 8.9604 $\mu$m, 8.9473 $\mu$m, or 8.7671 $\mu$m, and

in a case in which a concentration of the methanol is being measured, the concentration calculation unit calculates this concentration based on a methanol absorption of 9.5168 $\mu$m, 9.5042 $\mu$m, or 9.4861 $\mu$m.

4. The analysis device according to any one of Claims 1 through 3, wherein the concentration calculation unit calculates a concentration of the measurement target component by correcting an interference effect from an interference component other than the measurement target component that is contained in the processing gas.

5. The analysis device according to any one of Claims 1 through 4, wherein the laser light source is a quantum cascade laser.

6. The analysis device according to any one of Claims 1 through 5, further comprising a cell into which the processing gas is introduced, and
The cell is a multiple reflection cell or a resonance cell.

7. An analysis method in which a concentration of a measurement target component in the form of at least one of carbon dioxide, carbon monoxide, ethylene, ethane, water, acetylene, methane, ammonia, or methanol that are present in a processing gas is measured, wherein,

in a case in which a concentration of the carbon dioxide is being measured, this concentration is calculated based on a carbon dioxide absorption between 4.23 ~ 4.24 $\mu$m, or 4.34 ~ 4.35 $\mu$m,

in a case in which a concentration of the carbon monoxide is being measured, this concentration is calculated based on a carbon monoxide absorption between 4.59 ~ 4.61 $\mu$m,

in a case in which a concentration of the water is being measured, this concentration is calculated based on a water absorption between 5.89 ~ 6.12 $\mu$m,

in a case in which a concentration of the acetylene is being measured, this concentration is calculated based on an acetylene absorption between 7.56 ~ 7.66 $\mu$m, or 7.27 ~ 7.81 $\mu$m,

in a case in which a concentration of the methane is being measured, this concentration is calculated based on a methane absorption between 7.67 ~ 7.80 $\mu$m, or 8.10 ~ 8.14 $\mu$m,

in a case in which a concentration of the ethylene is being measured, this concentration is calculated based on an ethylene absorption between 8.46 ~ 8.60 $\mu$m,

in a case in which a concentration of the ethane is being measured, this concentration is calculated based on an ethane absorption between 6.13 ~ 6.14 $\mu$m, or 6.09 ~ 6.45 $\mu$m,

in a case in which a concentration of the ammonia is being measured, this concentration is calculated based on an ammonia absorption between 6.06 ~ 6.25 $\mu$m, or 8.62 ~ 9.09 $\mu$m, and

in a case in which a concentration of the methanol is being measured, this concentration is calculated based on a methanol absorption between 9.35 ~ 9.62 $\mu$m.

100

4

2
LASER LIGHT SOURCE

LASER LIGHT

1
CELL

LASER LIGHT

5
PHOTODETECTOR

TEMPERATURE ADJUSTMENT UNIT

3

6

OUTPUT SIGNAL

CONTROL SIGNAL

SIGNAL PROCESSING DEVICE

CONTROL SIGNAL

DETECTION TEMPERATURE SIGNAL

# Fig.1

OUTPUT SIGNAL FROM PHOTODETECTOR

6

SIGNAL PROCESSING UNIT

8

LOGARITHM CALCULATION UNIT — 81

STORAGE UNIT — 83

CORRELATION VALUE CALCULATION UNIT — 82

84

WAVELENGTH SHIFT DETERMINATION UNIT

CONCENTRATION CALCULATION UNIT — 85

CONTROL UNIT

7

RELATIONAL DATA STORAGE UNIT — 73

71

72

LIGHT SOURCE CONTROL UNIT

TEMPERATURE ADJUSTMENT CONTROL UNIT

CONTROL SIGNAL TO SEMICONDUCTOR LASER

CONTROL SIGNAL TO TEMPERATURE ADJUSTMENT UNIT

# Fig.2

Fig.3

Fig.4

MODULATION PERIOD T

OSCILLATION WAVELENGTH

LIGHT INTENSITY I (t)

log

LOGARITHMIC INTENSITY L (t)

×

CHARACTERISTIC SIGNAL F$_i$ (t)

∫

CORRELATION VALUE S$_i$

Time

# Fig.5

Fig.6

(a)

$$P(\Delta\lambda)=t_k(T-T_0)$$

PARAMETER P($\Delta\lambda$)

0

$T_0$

AMBIENT TEMPERATURE T(°C)

(b)

$$P(\Delta w)=v_k(T-T_0)$$

PARAMETER P($\Delta w$)

0

$T_0$

AMBIENT TEMPERATURE T(°C)

**Fig.7**

(a)

| AMBIENT TEMPERATURE T($^\circ$C) | T$_1$ | T$_2$ | T$_3$ | ... | T$_N$ |
|---|---|---|---|---|---|
| PARAMETER P($\Delta\lambda$) | P$_1$($\Delta\lambda$) | P$_2$($\Delta\lambda$) | P$_3$($\Delta\lambda$) | ... | P$_N$($\Delta\lambda$) |

(b)

| AMBIENT TEMPERATURE T($^\circ$C) | T$_1$ | T$_2$ | T$_3$ | ... | T$_N$ |
|---|---|---|---|---|---|
| PARAMETER P($\Delta$w) | P$_1$($\Delta$w) | P$_2$($\Delta$w) | P$_3$($\Delta$w) | ... | P$_N$($\Delta$w) |

**Fig.8**

Fig.9

6

OUTPUT SIGNAL FROM PHOTODETECTOR

SIGNAL PROCESSING UNIT

LOGARITHM
CALCULATION UNIT ⟋ 81

STORAGE
UNIT ⟋ 83

CORRELATION VALUE
CALCULATION UNIT ⟋ 82

8

86

BROADENING FACTOR
DETERMINATION UNIT

CONCENTRATION
CALCULATION UNIT ⟋ 85

CONTROL UNIT

RELATIONAL
DATA STORAGE
UNIT ⟋ 73

7

71

72

LIGHT SOURCE
CONTROL UNIT

TEMPERATURE ADJUSTMENT
CONTROL UNIT

CONTROL SIGNAL
TO
SEMICONDUCTOR
LASER

CONTROL SIGNAL
TO TEMPERATURE
ADJUSTMENT
UNIT

**Fig.10**

100

LASER
LIGHT

2
CELL

LASER
LIGHT

5
PHOTODETECTOR

4

2
LASER LIGHT
SOURCE

TEMPERATURE
ADJUSTMENT UNIT

3

OUTPUT SIGNAL

4

2
LASER LIGHT
SOURCE

TEMPERATURE
ADJUSTMENT UNIT

3

6

71
LIGHT SOURCE
CONTROL UNIT

4

2
LASER LIGHT
SOURCE

TEMPERATURE
ADJUSTMENT UNIT

3

72
TEMPERATURE
ADJUSTMENT
CONTROL UNIT

**Fig.11**

(A)

SPECTRUM WITHOUT COEXISTENCE EFFECTS

SPECTRUM WITH COEXISTENCE EFFECTS

(B)

SPECTRUM PRIOR TO PRESSURE CHANGES

SPECTRUM AFTER PRESSURE CHANGES

**Fig.12**

## INTERNATIONAL SEARCH REPORT

| International application No. |
|---|
| **PCT/JP2022/043508** |

**A. CLASSIFICATION OF SUBJECT MATTER**

*G01N 21/3504*(2014.01)i
FI:   G01N21/3504

According to International Patent Classification (IPC) or to both national classification and IPC

**B. FIELDS SEARCHED**

Minimum documentation searched (classification system followed by classification symbols)

G01N21/00-G01N21/61

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| Y | WO 95/026497 A1 (NIPPON SANSO CORPORATION) 05 October 1995 (1995-10-05) p. 7, line 15 to p. 16, line 4, fig. 1-4 | 1-7 |
| Y | JP 2002-507739 A (SCHLUMBERGER INDUSTRIES, S.A.) 12 March 2002 (2002-03-12) paragraphs [0081]-[0082] | 1-7 |
| Y | WO 2006/085646 A1 (JAPAN SCIENCE AND TECHNOLOGY AGENCY) 17 August 2006 (2006-08-17) paragraphs [0024], [0027] | 5-6 |
| Y | JP 2006-52955 A (JAPAN SCIENCE AND TECHNOLOGY AGENCY) 23 February 2006 (2006-02-23) paragraph [0008] | 6 |

☐ Further documents are listed in the continuation of Box C.        ☑ See patent family annex.

| * Special categories of cited documents: | |
|---|---|
| "A" document defining the general state of the art which is not considered to be of particular relevance | "T" later document published after the international filing date or priority date and not in conflict with the application but cited to understand the principle or theory underlying the invention |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **20 January 2023** | **07 February 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**INTERNATIONAL SEARCH REPORT**
Information on patent family members

| International application No. |
| --- |
| **PCT/JP2022/043508** |

| Patent document<br>cited in search report | | | Publication date<br>(day/month/year) | Patent family member(s) | | | Publication date<br>(day/month/year) |
| --- | --- | --- | --- | --- | --- | --- | --- |
| WO | 95/026497 | A1 | 05 October 1995 | US | 5703365 | A | |
| | | | | column 5, line 48 to column<br>11, line 41, fig. 1-4 | | | |
| | | | | US | 5821537 | A | |
| | | | | EP | 706042 | A1 | |
| | | | | KR | 96-0702607 | A | |
| | | | | TW | 315410 | B | |
| | | | | JP | 2587214 | B2 | |
| JP | 2002-507739 | A | 12 March 2002 | US | 6420695 | B1 | |
| | | | | column 7, lines 22-28 | | | |
| | | | | WO | 1999/049298 | A1 | |
| | | | | EP | 1068510 | A1 | |
| | | | | FR | 2776771 | A1 | |
| | | | | AU | 2844499 | A | |
| | | | | BR | 9908956 | A | |
| | | | | HU | 102276 | A2 | |
| | | | | PL | 343006 | A1 | |
| | | | | CA | 2325485 | A1 | |
| | | | | ID | 27588 | A | |
| | | | | CN | 1294679 | A | |
| WO | 2006/085646 | A1 | 17 August 2006 | US | 2009/0026374 | A1 | |
| | | | | paragraphs [0058], [0069] | | | |
| | | | | EP | 1850112 | A1 | |
| | | | | CA | 2597457 | A1 | |
| JP | 2006-52955 | A | 23 February 2006 | (Family: none) | | | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016090521 A